(19) Europäisches Patentamt / European Patent Office / Office européen des brevets

(11) **EP 2 872 030 B1**

(12) **EUROPEAN PATENT SPECIFICATION**

(45) Date of publication and mention
of the grant of the patent:
**07.12.2016 Bulletin 2016/49**

(21) Application number: **12735462.9**

(22) Date of filing: **10.07.2012**

(51) Int Cl.:
*A61B 3/10* (2006.01)    *A61B 3/00* (2006.01)

(86) International application number:
**PCT/EP2012/002906**

(87) International publication number:
**WO 2014/008904 (16.01.2014 Gazette 2014/03)**

(54) **PROCESS AND APPARATUS FOR DETERMINING OPTICAL ABERRATIONS OF AN EYE**

VERFAHREN UND VORRICHTUNG ZUR BESTIMMUNG OPTISCHER ABERRATIONEN EINES AUGES

PROCÉDÉ ET APPAREIL DE DÉTERMINATION D'ABERRATIONS OPTIQUES DANS UN IL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.05.2015 Bulletin 2015/21**

(73) Proprietor: **WaveLight GmbH**
**91058 Erlangen (DE)**

(72) Inventors:
• **WUELLNER, Christian**
**91094 Bräuningshof (DE)**

• **DONITZKY, Christof**
**90542 Eckental/Eschenau (DE)**
• **KAEMMERER, Maik**
**90614 Ammerndorf (DE)**

(74) Representative: **Frenkel, Matthias Alexander**
**Wuesthoff & Wuesthoff**
**Patentanwälte PartG mbB**
**Schweigerstrasse 2**
**81541 München (DE)**

(56) References cited:
**WO-A1-2010/109020    US-A1- 2003 117 581**

**Description**

An embodiment of the invention relates to a process and to an apparatus for determining optical aberrations of an eye, including the optically active constituents of the eye.

It is known to determine wavefront aberrations of an eye according to deviations of a wavefront resulting from an optical system of the eye with respect to a planar wavefront generated using an aberration-free eye model. The wavefront resulting from an optical system may be determined using a Tscherning aberrometer, a Hartman-Shack aberrometer or a digital wavefront sensor.

A Tscherning aberrometer is an apparatus, known as such, for measuring the aberrations of an eye. According to the principle of Tscherning aberrometry, a plurality of component beams are generated from parallel light. The generated component beams exhibit a predetermined two-dimensional arrangement in a plane arranged perpendicular to an optical axis. This arrangement of the component beams is projected through the optical system of the eye, and a first pattern of dots with first projections of the component beams is thereby generated on the retina of the eye as retinal points of light. The first pattern of dots generated on the retina is projected in the reverse direction through the optical system of the eye into a second plane arranged outside the eye, and a second pattern of dots with second projections (second points of light) of the first projections of the component beams generated on the retina is generated by this projection. The second pattern of dots is recorded as an image. Offsets between each second point of light and a projection generated with an aberration-free eye model of the same component beam of the regular two-dimensional arrangement are measured in the image plane. From the plurality of the measured offsets, deviations of a real wavefront generated by the optical system of the eye from the ideal planar wavefront of an ideal, imaging-error-free (aberration-free) eye are reconstructed. From the reconstructed wavefront aberrations of the real eye, the imaging errors of the eye, inclusive of the higher-order imaging errors, can be determined. Regarded as higher-order imaging errors are, for example, coma, trefoil, spherical aberration, quadrafoil, etc. An ocular aberrometer (e.g., Tscherning aberrometer) and a process for utilising the aberrometer, as well as a process for evaluating the second pattern for evaluating the offsets measured in the second pattern of the points of light stemming from the component beams are known in the current art.

The wavefronts of the eye, determined e.g. with the Tscherning aberrometer, that include imaging errors may serve as a basis - together with measurements of the eye, such as the corneal thickness, the anterior-chamber thickness, the lens thickness and the ocular length as well as the topographies of the anterior corneal surface and of the posterior corneal surface, obtained, for example, from OCT (optical coherence tomography) measurements or Scheimpflug recordings - in order to create an eye-specific eye model. From an individual eye model created in this way e.g. an ablation profile for a laser-surgical refraction correction of the anterior corneal surface of the eye and/or a design, and/or a front lens face, for an intraocular lens to be inserted surgically into the eye for the purpose of correcting imaging errors of the optical system of the eye can then be computed.

Processes known hitherto for computing the individual wavefronts of an eye that, due to the imaging errors of the optical system of the eye, differ in their shape from an ideal planar wavefront are based on model assumptions for the biometric data of the eye, e.g., the ocular length. This applies to aberrometers that operate according to the Tscherning principle and also to instruments that utilise a Hartmann-Shack sensor or a digital wavefront sensor.

In known instruments the Gullstrand eye model, for example, with its ocular length of 22.36 mm, serves as a basis. The present embodiment of the invention is derived from the finding, based on computational simulations and clinical experience from applications of the aberrometer and other devices for surveying the optical system of an eye show: the more the actual ocular length differs from the model assumption for the ocular length, the greater the error in the defective vision of the eye computed from wavefront measurements. This is illustrated in the following table. Therein, for five ocular lengths differing from the model assumption (22.36 mm) for the ocular length, the differences of the wavefront, expressed in spherical and cylindrical aberrations of the defective vision, have been computed and compiled:

| | Result of Measurement | | Difference from the Model | |
|---|---|---|---|---|
| Ocular Length [mm] | Sphere [dpt] | Cylinder [dpt] | Sphere [dpt] | Cylinder [dpt] |
| 20.36 | - 5.17 | 0.36 | - 0.42 | 0.03 |
| 21.36 | - 4.95 | 0.35 | - 0.20 | 0.02 |
| 22.36 (Gullstrand) | - 4.75 | 0.33 | 0.00 | 0.00 |
| 23.36 | - 4.57 | 0.32 | 0.18 | -0.01 |
| 24.36 | - 4.39 | 0.31 | 0.36 | -0.02 |
| 25.36 | - 4.24 | 0.30 | 0.51 | - 0.03 |

A process and an apparatus according to the preambles of claims 1 and 13 is disclosed in document US 2003/117581.

An object of certain embodiments is to make available certain processes and an apparatuses for determining wavefront aberrations of an eye that enable a more accurate computation or characterisation of the defective vision, inclusive of the higher-order aberrations. The embodiments permit an optimised individual eye model as well as an optimised ablation profile for a laser-surgical refraction correction of the cornea and/or an intraocular lens to be computed with regard to shape and material.

The object is achieved by a process for determining optical aberrations of an eye in accordance with claim 1.

In accordance with an embodiment of the invention, the process comprises reconstructing wavefront aberrations of the eye as a deviation of the wavefront, determined by the optical system of the eye with a process of aberrometry, with respect to an ideal planar wavefront generated by an aberration-free improved eye model. Furthermore, in the eye model the actual ocular length is used as the model ocular length.

The actual ocular length can be determined for this purpose from a biometric measurement of the ocular length carried out in respect of the eye. Measurements of the actual ocular length on the optical axis (i.e. visual axis) of the eye can be carried out with instruments according to the latest known art. The WaveLight OB 820 instrument may be mentioned as an example of these instruments.

The use of the actually measured ocular length in the eye model makes it possible that wavefront aberrations and the imaging errors of the optical system of the eye, derived from the results of the aberrometric measurements, may be computed with higher accuracy and may improve treatment outcomes of wavefront-guided refractive corneal surgery. Furthermore, the computed wavefront aberrations and the imaging errors may improve intraocular lenses inserted into an eye to correct the imaging errors.

In accordance with the inventive determination of the optical aberration of an eye, the anterior surface of the cornea or an intraocular lens (IOL) can be modified to correct presbyopia.

An embodiment of the invention is also applied in order to correct presbyopia by specific induction of aberrations up to the 4th Zernike-order (cylinder, coma, trefoil, quadrafoil and spherical aberrations) in order to achieve better close vision.

The process may comprise the following steps:

  (a) generating a plurality of individual component beams of a bundle of light rays of parallel light, with a two-dimensional arrangement of the component beams,
  (b) projecting the arrangement of the component beams through the optical system of the eye and thereby generating a first pattern of dots with first projections of the component beams on the retina of the eye;
  (c) ophthalmoscopically projecting the first pattern into a second plane arranged outside the eye, and thereby generating a second pattern of dots from second projections of the first projections of the component beams generated in step (b);
  (d) recording the second pattern in the second plane;
  (e) constructing by calculation an aberration-free eye model with a model ocular length for which the measured actual ocular length is used by way of value;
  (f) for each second projection of a component beam, recorded in the second plane, measuring an offset of the ophthalmoscopic projection of the component beam in the second pattern of dots with respect to a projection, generated with the constructed eye model, of the same component beam of the two-dimensional arrangement; and
  (g) reconstructing the wavefront aberrations of the optical system of the eye as deviations of the wavefront generated by the optical system with respect to a wavefront generated by the aberration-free eye model.

The eye model may represent the eye for the purpose of characterising the imaging properties of the eye within the scope of predetermined tolerance limits with regard to predetermined quality parameters.

These techniques for constructing the improved, aberration-free eye model, for measuring the offset, and for reconstructing the wavefront aberrations of the optical system of the eye can be used. In certain cases, the wavefront aberrations can be computed more accurately by replacing the model assumptions for the ocular length by the actually measured value of the ocular length.

The two-dimensional arrangement of the component beams may be matrix-shaped in a first plane arranged substantially perpendicular to an optical axis of the eye.

If the optical axis of the eye is called the first optical axis the second plane may be arranged substantially perpendicular to a second optical axis that is angled with respect to the first optical axis of the eye. The projection of the first pattern into the second plane can be performed by means of instruments for indirect ophthalmoscopy.

The offset of the second projections of the component beams in the second plane is, for example, measured along two coordinate axes (x, y) which are mutually perpendicular in the second plane. In this case an offset in the x-direction and an offset in the y-direction can be measured.

The improved, aberration-free eye model with the actual ocular length can, for example, be constructed using the Gullstrand eye model or the Liou-Brennan eye model. These two eye models mentioned above have proved their worth in examinations, undertaken by the applicant, of wavefront aberrations on the basis of model assumptions for the ocular length. Surprisingly, it has been found that introducing the actual ocular length in place of the model ocular lengths used hitherto in these models makes possible the improvements - far exceeding expectations - in the accuracy of the computed wavefront aberrations and of the imaging errors derived therefrom (aberrations of lower and higher order) of an individual eye. The use of the actual ocular length also yields improved results for eye models other than those named above.

The actual ocular length can be determined from a direct measurement of the ocular length of the eye of the patient. In this case the WaveLight OB 820 instrument already mentioned above may be employed, for example.

The measurement of ocular length can be performed at any suitable point, such as immediately before one of steps (a) to (e). The measurement of ocular length can also be performed using at least a part of the parallel bundle of light rays used in step (a).

In the case of the projection of the arrangement of the component beams through the optical system of the eye, in accordance with step (b) of the process a focal point of the projection may be situated a predetermined distance upstream of the retina of the eye.

In step (g) in the course of the improved reconstructing of the wavefront aberrations the wavefront generated by the optical system of the eye can be represented as a sum of Zernike polynomials normalised to a unit circle and weighted with corresponding Zernike polynomial coefficients. In this case the reconstructing of the wavefront generated by the optical system of the eye may include the determination of the Zernike polynomial coefficients of the Zernike polynomials used for the purpose of representing the wavefront. In the stated sum of the Zernike polynomials the Zernike polynomials can be taken into account as far as the 6th order, and as far as the 8th order. From the determined Zernike polynomial coefficients a spherical refraction of the eye, a cylindrical refraction of the eye and an angle of an astigmatism of the eye can be computed from the Zernike polynomial coefficients for the third, fourth and fifth Zernike polynomial (i.e. from the Zernike polynomials of 2nd order) and also from the radius of the pupil of the eye.

On the basis of the reconstructed wavefront aberrations improved in step (g), an improved patient-specific eye model can be created. The improved patient-specific eye model can furthermore be created on the basis of the following:

(1) a corneal thickness, an anterior-chamber depth, a lens thickness and/or lens position and the actual ocular length of the eye, which in each instance have been determined from measurements carried out in respect of the eye;
(2) a topography of the anterior corneal surface and a topography of the posterior corneal surface, which in each instance have been obtained from measurements carried out in respect of the eye; and
(3) a front lens face and a back lens face, which in each instance have been acquired from an iterative computation, utilising an optical ray-tracing process and with the data stated under points (1) and (2).

In this case, the data stated under point (1) can be determined on the optical axis (e.g. visual axis) of the eye.

The measurements stated under point (2) and carried out in respect of the eye may be interferometric measurements, such as measurements using OCT (optical coherence tomography). The topographies of the anterior corneal surface and of the posterior corneal surface can be acquired, for example, with Scheimpflug measurements. OCT measurements can also be employed for this.

The patient-specific eye model may be used to generate an optimised ablation profile for a laser-surgical refraction correction of the pre-operative anterior corneal surface of the eye or a design, such as a front lens face design, for an intraocular lens to be surgically inserted into the eye for the purpose of correcting imaging errors of the optical system of the pre-operative eye.

According to a second aspect of an embodiment of the invention, an apparatus is made available for determining optical aberrations of a human eye, with its optical system including the cornea and the lens. The apparatus comprises: the features of claim 13.

In accordance with an embodiment of the invention, the apparatus includes an arithmetic unit, for example a computer, with:

means for constructing an improved, aberration-free eye model with an ocular length, and
means for improved reconstructing of wavefront aberrations of the eye as a deviation of the wavefront determined by the optical system of the eye with the aberrometer with respect to an ideal planar wavefront generated by the improved eye model.

Furthermore, in accordance with an embodiment of the invention, the means for constructing the improved eye model are designed to use an actual ocular length by way of value for the model ocular length. The apparatus according to the second aspect of one embodiment of the invention achieves the same advantages as the process, described above, according to the first aspect.

The improved eye model may represent the eye for the purpose of characterising the imaging properties of the eye within the scope of predetermined tolerance limits with regard to predetermined quality parameters.

In one example, a Tscherning aberrometer may include, for example, the following:

a light-source for generating a bundle of light rays consisting of parallel light,
a device for generating a plurality of component beams from the bundle of light rays with a two-dimensional arrangement of the component beams,
an aberroscope lens, arranged on the first optical axis, for projecting the arrangement of the component beams through the optical system of the eye and thereby generating the first pattern of dots with the first projections of the component beams onto the retina of the eye,
an ophthalmoscopic device for ophthalmoscopically projecting of the first pattern of dots into a second plane arranged outside the eye, to yield a second pattern of dots with second projections of the first projections.

The arithmetic unit may include means for measuring, for each second projection of a component beam recorded with the ophthalmoscopic device, an offset of the second projection in the second plane with respect to a projection, generated with the improved eye model, of the same component beam of the two-dimensional arrangement. For said measurement, a measuring device can be used or any other subject matter performing the defined function.

In the aberrometer the two-dimensional arrangement of the component beams in a first plane arranged substantially perpendicular to a first optical axis (e.g. visual axis) of the eye may be regular, and/or two-dimensionally regular, for example matrix-shaped.

The ophthalmoscopic device may exhibit a second optical axis, angled with respect to the first optical axis, and the second plane may be arranged substantially perpendicular to the second optical axis.

The offset of the second projections can be measured along two mutually perpendicular coordinate axes (x, y) arranged in the second plane, so that consequently for each second projection of a component beam an offset in the x-direction and an offset in the y-direction can be measured.

The ophthalmoscopic device may further include the following:

a first partially transmitting mirror arranged substantially diagonally between the aberroscope lens and the eye with respect to the first optical axis for the purpose of deflecting the beam path extending from the retina through the optical system of the eye and thereby forming the second optical axis,
an ophthalmoscope lens arranged on the second optical axis for the purpose of projecting the first pattern and, by virtue of the projection, generating the second pattern with the second projections of the first projections of the component beams generated on the retina, and
a recording device arranged at the termination of the second optical axis for recording the second pattern of the second projections.

The recording device may be a CCD camera, which may be equipped with a highly light-sensitive CCD.

The ophthalmoscopic device may further include a diaphragm for defining an optical channel that is axially close with respect to the second optical axis. The diaphragm may be arranged between the ophthalmoscope lens and the recording device.

The light-source may exhibit a laser for generating a laser beam and optics for expanding the laser beam and for generating the bundle of light rays. The laser may be an IR laser or, for example, a laser emitting in the visible (red) region. For the stated optics, a Keplerian telescope device, for example, may be configured.

The apparatus may further an eye-aligning device which exhibits a third optical axis, angled with respect to the first optical axis, and which includes the following:

a second partially transmitting mirror, arranged substantially obliquely with respect to the first optical axis, for developing the third optical axis,
a converging lens arranged on the third optical axis for projecting the anterior portion of the eye, and
a second recording device arranged on the third optical axis for recording an image of the anterior portion of the eye.

The second partially transmitting mirror may be arranged between the light-source and the aberroscope lens.

The eye-aligning device may be designed to operate in the infrared region of the light spectrum. For this purpose the second partially transmitting mirror can deflect light emerging from the eye in the infrared region. The second recording device may for this purpose be a CCD camera with an infrared-sensitive CCD sensor. Furthermore, an infrared band-pass filter may be arranged on the third optical axis between the second partially transmitting mirror and the second ophthalmoscope lens.

The two-dimensional arrangement of the component beams can be generated by an aperture mask, arranged in the bundle of light rays, with a plurality of holes or light-transmission apertures provided therein. An arrangement of the holes is may be a two-dimensionally regular arrangement, for example a regular arrangement with a two-dimensionally rectangular or square structure which is formed in each instance by adjacent holes. Such a regular arrangement of the component beams makes it possible that, in the first and second patterns of dots projected through the optical system of the eye, deviations of the pattern of dots or of the position of individual second projections from the regular arrangement are already readily perceptible visually and an experienced ophthalmologist can make an estimation, already based on the visual impression, of the type of defective vision of the eye.

Further possibilities for configuring the process or the apparatus according to embodiments of the invention result from the following detailed description of embodiments. These will be described with reference to the appended drawings, in which:

Fig. 1    shows schematically a basic configuration of an example of Tscherning aberrometer;

Fig. 2    shows schematically an embodiment of an optical aberrometer for clinical use;

Fig. 3    shows, in the left-hand part, an example of a design of the two-dimensional regular arrangement of component beams, in the middle and right-hand parts, two examples of processed and inverted reproduction, generated by an ophthalmoscopic projection into an image plane arranged outside the eye, of patterns of dots generated on the retina, and, in the right-hand part, and also magnified in the lower part, the principle of the evaluation of the second pattern of dots by measuring the offsets of the second projections;

Fig. 4    shows schematically the eye model according to Gullstrand, in the upper part the geometrical arrangement of the optical elements of the model eye, and in the lower part in the table the numerical values of the optical properties of the optical elements;

Fig. 5    shows schematically the Liou-Brennan eye model, in the upper part the geometrical arrangement of the optical elements of the model eye, and in the lower part in the table the numerical values of the optical properties of the optical elements;

Fig. 6    shows schematically an example of a projection, generated with an aberration-free eye model, of the component beams of the two-dimensional regular arrangement, by which an ideal (planar) wavefront is generated, and a real wavefront generated by a projection of the same component beams burdened by imaging errors (aberrations), and the definition of wavefront aberrations as deviations between the real and the ideal wavefronts;

Fig. 7    shows schematically an example of the beam path of the aberrometer from Fig. 2 on the eye (cornea and retina) with an aberration lens arranged upstream of the eye;

Fig. 8    shows schematically an example of the beam path of the aberrometer from Fig. 2 in a strongly myopic eye (cornea and retina) without aberration lens;

Fig. 9    shows schematically an example of a configuration of an improved, individualised eye model on the basis of measured data, and beams of light tracked in an iterative optimisation process based on optical ray tracing;

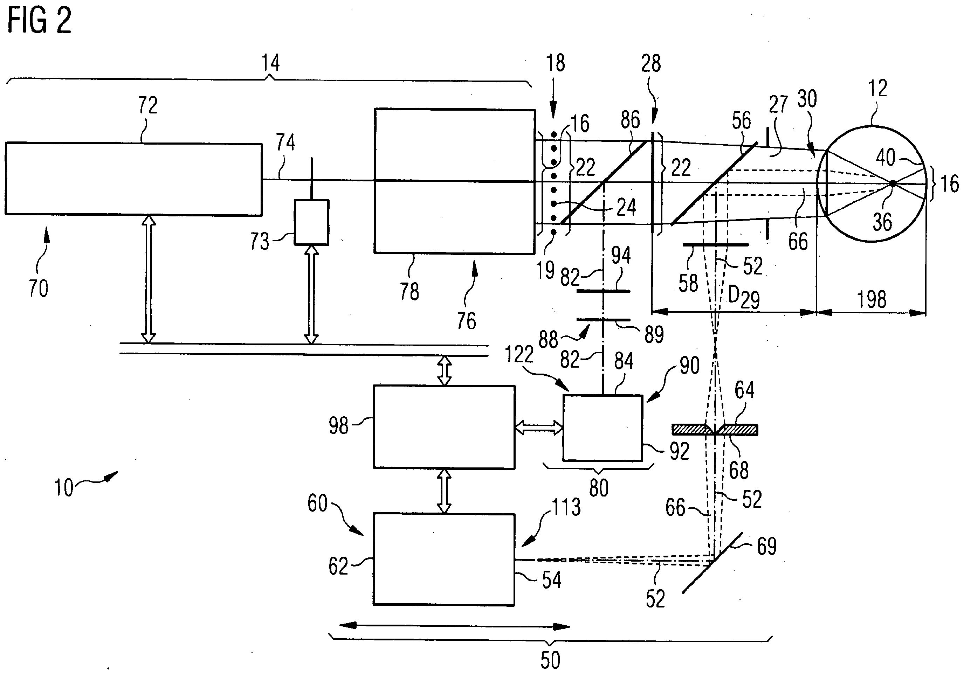

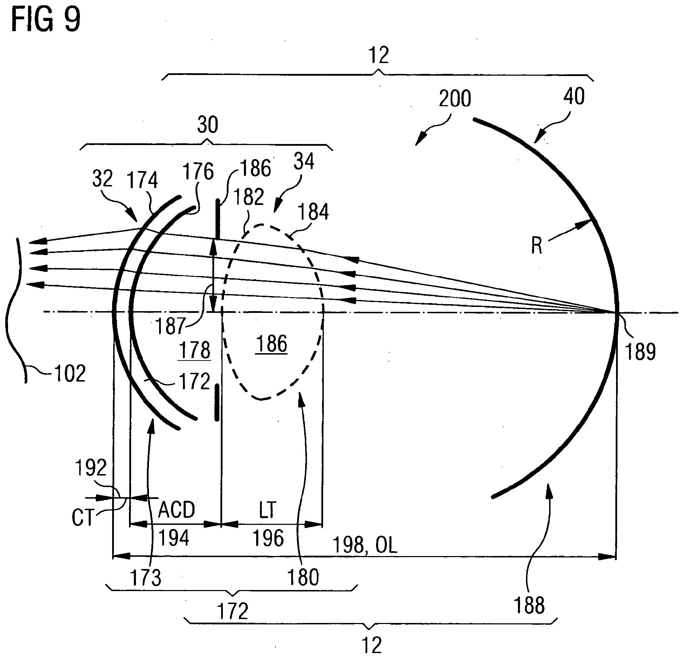

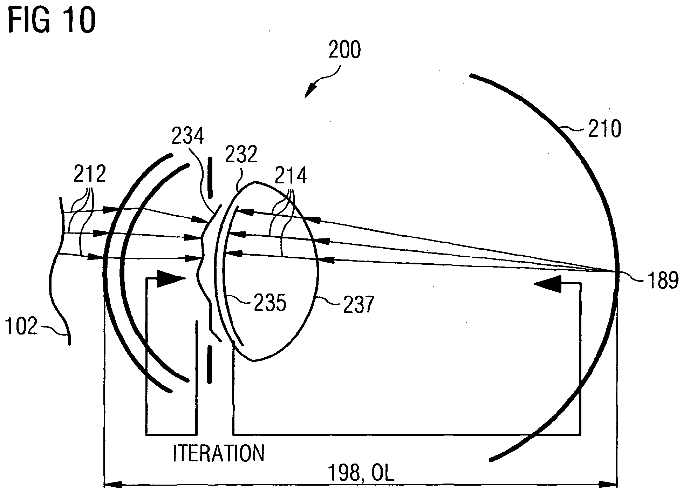

Fig. 10   shows schematically an example of an individualisation process for the improved eye model from Fig. 9, wherein after an implementation of the measured topography of the anterior corneal surface and of the posterior corneal surface, and also of the lengths of the cornea, of the anterior chamber, of the lens and of the eye, an intraocular optical surface, such as, for instance, the front lens face, is optimised up until the point where the simulated overall wavefront of the eye is equal to the wavefront aberration determined with the aberrometer from Fig. 2, and the improved optimisation process is performed iteratively from two sides of the surface to be optimised;

Fig. 11   shows an example of individualised eye model of a patient after the adaptation of the intraocular surfaces (for example, of the front lens face, as shown in Fig. 10), wherein a bundle of light rays emanating from the retina is tracked through the entire eye, so that upstream of the improved eye model the reconstructed wavefront aberration coincides with the measured one;

Fig. 12    shows schematically how an example of an ablation profile is derived from the difference between a pre-operative topography of the anterior corneal surface and the computed ideal anterior corneal surface;

Fig. 13    shows schematically how an example of a front face of an intraocular lens can be optimised by presetting a target wavefront that can be derived from the improved wavefront aberrations determined with the aberrometer from Fig. 2.

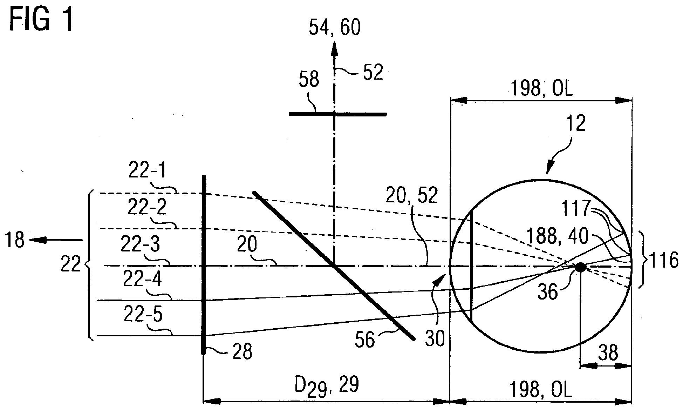

Fig. 1 illustrates the optical principle of an aberroscope due to Tscherning, for example according to the embodiment for clinical use shown in Fig. 2, with a first optical axis 20 (e.g. visual axis) of the eye 12 and with a second optical axis 52 of an ophthalmoscope. In this case, in the eye 12 shown in Figures 1 and 2 the optical system 30 comprising the cornea and the lens is summarised schematically as an active optical element. The aberroscope includes an aberroscope lens 28, a first partially transmitting mirror 56 and an ophthalmoscope lens 58. A plurality of individual thin parallel beams of light, which in the representation shown in Fig. 1 are incident from the left and which form a two-dimensionally regular matrix in a plane arranged perpendicular to the first optical axis 20, are focused onto the eye 12 by the aberroscope lens 28 with a relatively low refractive power, arranged upstream of the eye 12, in such a way that in the eye an intraocular focal point 36 arises which is situated a predetermined distance 38 upstream of the retina 40 of the eye 12. By virtue of the aberroscope lens 28, the regular two-dimensional arrangement of the component beams 22, 22-1, ..., 22-5 is projected through the optical system 30 of the eye onto the retina 40 and generates there a first pattern of dots 116 consisting of individual, first projections 117 of the component beams 22.

The first pattern of dots 116 projected onto the retina 40 can be perceived subjectively by the patient. The patient could write down the shape of the perceived pattern of points of light subjectively and at least qualitatively. As regards the arrangement of the points of light, the perceived pattern of points of light differs in its shape from the regular shape of the regular two-dimensional arrangement of the component beams 22-1, ..., 22-5 beamed in from the left in Figures 1 and 2 by reason of the projection, burdened with imaging errors (aberrations), through the optical system 30 of the eye.

The two upper component beams 22-1 and 22-2 (dotted lines) shown in Fig. 1 show the ideal case of a projection through the optical system 30 of the eye without any monochromatic optical aberrations. These "ideally" projected component beams 22-1 and 22-2 pass through the focal point 36 of the projection. The two lower component beams 22-4 and 22-5 shown in Fig. 1 show a situation in which the optical system 30 of the eye exhibits aberrations. These component beams 22-4 and 22-5 projected with imaging errors undergo varying refractive powers, depending on the position of their point of intersection through the optical system 30 of the eye. The points of light, i.e. the projections 117 of the component beams 22 burdened with imaging errors and projected onto the retina 40, that together form the first pattern of dots 116 are deflected, by means of indirect ophthalmoscopy via a first partially transmitting mirror 56, out of the first optical axis 20 (e.g. visual axis) of the eye in the direction of a second optical axis 52 and projected, by means of an ophthalmoscope lens 58 and a narrow optical channel 66 defined by a diaphragm 64 (see also Fig. 2), into a second plane 54 arranged outside the eye and arranged substantially perpendicular to the second optical axis 52.

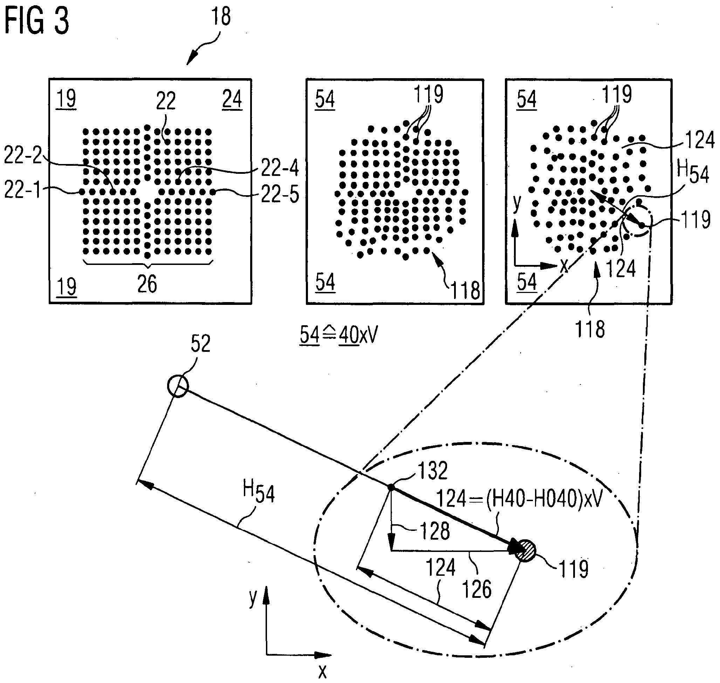

According to Fig. 3, an image sensor of an image-recording device 60 is arranged for the purpose of recording the second projections 119 generated by the ophthalmoscopic projection in the second plane 54.

In an eye with ideal optical properties, i.e. without any optical aberrations, this subjectively discernible pattern of dots would exhibit the same two-dimensional regularity as the component beams 22-1, ..., 22-5 coming from the left in Fig. 1 and arriving at the aberroscope lens 28. For a patient's eye 12 burdened with imaging errors (optical aberrations), the first pattern of dots of light 116 generated on the retina 40 is more or less distorted by the imaging errors (aberrations) of the optical system 30 of the eye 12.

In order to quantitatively measure the distortion of the first pattern of dots of light 116 generated on the retina 40, the positions of the individual first projections 117, generated on the retina 40, of the individual component beams 22-i (i = 1, ..., 5) (the so-called retinal points of light) are recorded and their offset with respect to an aberration-free projection is determined. For this purpose, the first pattern of dots 116 generated on the retina 40 is projected by an ophthalmoscopic device 50 including the first partially transmitting mirror 56 and the first ophthalmoscope lens 58 into the second plane 54 arranged of the eye 12. The second plane 54 is, for example, the image plane of a sensor of an image-recording device 60, such as, a video camera. So in the second plane 54 a second pattern of dots 118 is generated with the second projections 119, generated by the ophthalmoscope lens 58 (see Fig. 3, middle and right-hand parts), of the first projections 117 (retinal points of light), arranged on the retina 40, of the first pattern of dots 116. In the image of the second pattern of dots 118 recorded in the second plane 54, the coordinates of the geometrical midpoints of the second projections 119 are determined by means of computer-assisted image-processing software.

The distortion of the second pattern of dots 118 with respect to the originally regular two-dimensional arrangement 26 of the component beams 22-1, ... , 22-5 (see Fig. 3, left-hand part) can be described quantitatively. For each point of light - i.e. for each projection 119 of a point of light 117 of the retinal, first pattern of dots 116 - an offset 124 is determined with respect to the "ideal" point of light 132, assigned to the same component beam and obtained under aberration-free conditions. The shape of the arrangement of the ideal points of light 132 corresponds to the shape of the two-dimensional arrangement 26 of the component beams. For the positions of the ideal points of light 132, reference coordinates are computed for the case of an assumed, imaging-error-free (aberration-free) projection of the arrangement 26 of the component beams by an optical system with an equivalent mean spherical refraction assigned to the refractive power of the optical system 30. By virtue of the refraction, an ideal point of light 132 in the second plane 54 is assigned to each component beam 22 of the two-dimensional arrangement 26 of the component beams, beamed into the aberrometer. In the second plane 54, i.e. the image plane, an orthogonal coordinate system with two mutually perpendicular axes x and y is defined, see Fig. 3, middle and right-hand parts. Accordingly, the offset 124 of each second projection 119 with respect to the coordinates of the associated, ideal point of light 132 is represented as the vector sum of an offset 126 in the x-direction and an offset 128 in the y-direction; see the magnified representation in the lower region of Fig. 3.

The embodiment shown in Fig. 2 of an optical aberrometer (apparatus 10) is based on the Tscherning aberrometry, illustrated in Fig. 1 . The apparatus 10 for determining optical aberrations of a human eye 12 has been developed for clinical applications and includes a light-source 14 for generating a bundle of light rays 16 consisting of parallel light, a device 18 for generating a plurality of component beams 22 from the bundle of light rays 16 with the two-dimensional arrangement 26 (shown in Fig. 3), an aberroscope lens 28 for projecting the arrangement 26 through the optical system 30 of the eye 12 and thereby generating a first pattern of dots 116 on the retina 40 of the eye, an ophthalmoscopic device 50 for projecting the first pattern of dots 116 on the retina into a second plane 54 arranged outside the eye and thereby generating a second pattern of dots 118, and also a computer 98 with an arithmetic unit.

The light-source 14 includes a laser 70 which generates a laser beam 74, a controllable shutter device 73 for switching on and off or modulating the laser beam 74, a collimator device 76 for expanding the laser beam 74 and for generating an expanded bundle of light rays 16 consisting of parallel light. The laser 70 is a laser diode emitting in the red light region (approx. 660 nm) with a beam diameter of about 2 mm and with a power output of about 10 mW. The laser beam 74 is controlled by the electromechanical shutter device 73, so that an opening-time within the range from about 10 ms to 100 ms, or about 60 ms, is generated. By way of collimator device 76, use is made of an arrangement based on the principle of a Keplerian telescope 78, which widens the laser beam to a diameter of about 25 mm and exhibits a spatial filter (not shown) with a diameter of 15 $\mu$m. A mask (aperture mask) 19 serves as device 18 for generating the component beams 22. The aperture mask 19 exhibits a plurality of transmission apertures which are distributed in the aperture mask 19 in a regular, two-dimensional arrangement. Thus in a first plane defined by the device 18 (aperture mask 19), which is arranged substantially perpendicular to the first optical axis 20 (i.e. visual axis) of the eye, a matrix-shaped pattern of dots, as shown in the left-hand region of Fig. 3. The diameter of a component beam 22 is limited by the hole- diameter of the individual transmission apertures in the aperture mask 19 and amounts in the apparatus to about 0.2 mm to 0.5 mm, or about 0.33 mm. The aperture mask 19 takes the form of a photographic film. It is interchangeable, and several different aperture masks are made available, so that it can be ensured that for a respective, likewise interchangeable aberroscope lens 28 (as explained further below) such a mask 19 is available, so that a spacing of the component beams 22 on the anterior side of the cornea 32 of about 0.6 mm is generated. The various aperture masks 19 differ with regard to the dot spacing. The two-dimensional arrangement in the various dot masks 19 is congruent to one another.

In certain embodiments, the aberroscope lens 28 is interchangeable, and various aberroscope lenses 28 with varying refractive powers are made available. In practice it may be useful to displace the aberroscope lens. Interchangeable aberroscope lenses having varying refractive powers may also be employed, depending on the mean spherical refractive power of the eye. From the specification that the intraocular focal point 36 is to be situated a predetermined distance 38 upstream of the retina, it follows that the refractive power of the aberroscope lens 28 depends on the mean spherical refractive power of the optical system 30 (cornea and lens) of the eye 12. For the case of an emmetropic eye, the refractive power of the lens 28 is chosen within a range from about +4D to +5D. For a hyperopic eye with a refractive power of more than +2D, an aberroscope lens 28 with a refractive power of more than +5D is employed. The purpose of the aberroscope lens 28 consists in causing the first pattern of dots 116 generated on the retina 40 to be formed always in the same size in such a manner that the individual points of light 117 are separated from one another and easily identifiable. The retinal first pattern of dots 116 has a dimension of about 1 mm$^2$.

The eye 12 with its visual axis is correctly positioned on the first optical axis 20 of the bundle of component beams 22-1,..., 22-5 generated by the light-source 14 by means of an eye-aligning device 80 operating in the near infrared. The eye-aligning device 80 includes two diodes emitting in the near infrared (LEDs, not shown, emission wavelength: approx. 880 nm), which are aligned vertically with respect to the eye. The eye 12 is observed by a device likewise operating in the infrared region. Said device includes a second partially transmitting mirror 86 partially reflecting in the infrared region, a second recording device 90 operating in the infrared region, with a CCD video camera 92 with a CCD sensor (not shown) that is sensitive in the infrared region. The second partially transmitting mirror 86 is arranged substantially diagonally with respect to the first optical axis 20, with an angle of approximately 45° between the component-beam-generating device 18 and the aberroscope lens 28. The CCD sensor is arranged in a third plane 84 which is arranged substantially perpendicular to the third optical axis 82 deflected by the second partially transmitting mirror 86. For IR light the mirror 86 deflects the first optical axis 20 (e.g. visual axis) of the eye 12 into a second ophthalmoscope lens 88 arranged on a third optical axis 82, which takes the form of an infrared (IR) lens 89 (i.e. a lens transmitting in the infrared region), and into an infrared band-pass filter 94 adapted to the emission frequency of the infrared light-sources. For the purpose of blocking light in the visible spectral region, filter 94 is arranged on the third optical axis 82 between the second partially transmitting mirror 86 and the second ophthalmoscope lens 88 (i.e. infrared lens 89).

By means of the eye-aligning device 80 the eye is observed via the mirror 86 through the aberroscope lens 28 and the initial aperture of the aberroscope. The second partially transmitting mirror 86, the lens 88 and the second recording device 90 are adjusted, fixed with respect to the first optical axis 20 of the bundle of light rays 16 generated by the collimator device 76, in such a way that the third optical axis 82 coincides with the first optical axis 20 transmitted by the mirror 86. The correctly adjusted position of the eye 12 is then found and is fixed if the narrow bundle of light rays of the slit lamp and the initial aperture of the aberroscope on a monitor to which the infrared image recorded by the second recording device 90 is supplied are centred on the pupil of the eye 12 displayed on the monitor. The head of the patient is fixed for the correctly adjusted position of the eye 12 on a known art chin-rest (not shown) and by means of a known art forehead-stop (likewise not shown).

The first pattern of dots 116 generated by the bundle of the component beams 22 on the retina 40 of the eye 12 is recorded by means of a first ophthalmoscopic device 50. The ophthalmoscopic device 50 includes a first partially transmitting mirror 56 which is arranged on the first optical axis 20 substantially diagonally, i.e. with an angle of approximately 45°, between the aberroscope lens 28 and the initial aperture 27 of the aberroscope, a first ophthalmoscope lens 58, a diaphragm 64, and by way of first recording device 60 a CCD camera 62 with a CCD sensor, the image plane of which, the second plane 54, in which the second pattern of dots 118 generated by way of image of the first pattern of dots 116 generated on the retina by the projection through the optical system 30 of the eye 12 and the ophthalmoscope lens 58 is projected. In certain embodiments, the beam path (the second optical axis 52) is deflected by a deflecting mirror 69. The positions of the first recording device 60 (the CCD camera 62), the ophthalmoscope lens 58 and the deflecting mirror 69 are fixed relative to one another, to define the fixing a second optical axis 52 of the ophthalmoscopic device 50. The position or orientation of the ophthalmoscopic device 50 or the second optical axis 52 with respect to the first partially transmitting mirror 56 is adjusted in such a way that the first optical axis 20 deflected by the mirror 56 coincides with the second optical axis 52 of the ophthalmoscopic device 50.

The ophthalmoscopic device 50 includes a diaphragm 64 arranged immediately upstream of the camera lens 68, the opening of which defines an optical channel 66. The optical channel 66 may have a narrow diameter and may be defined axially closely along the first optical axis 20 (e.g. visual axis) of the eye 12. Channel 66 defines the second optical axis 52 of the ophthalmoscopic device 50 deflected by the mirror 56 in axially close manner. The narrow diameter of the optical channel 66 pierces only a correspondingly small, axially close region of the optical system 30 of the eye 12, so that the ophthalmoscopic projection may be regarded as virtually free from imaging errors.

The ophthalmoscope lens 58 projects the retinal, first pattern of dots 116 together with the camera lens 68 (focal length, e.g., 30 mm) through the narrowly limited optical channel 66 onto the CCD sensor array (in the example with a dimension of 12.8 mm x 9.6 mm) of the CCD video camera 62 (e.g., type LH 750LL, Lheritier S.A., Cergy Pontoise, France, or Watec WAT-902H2). The position of the camera is adjustable to inspect various partial regions of the retinal intermediate image (of the first pattern of dots 116), depending on the refractive power of the eye 12. For the purpose of recording the second pattern of dots 118 no optical correction of the optical system 30 of the eye is undertaken.

The image of the second pattern of dots 118 recorded by the first recording device 60 is transmitted to the computer 98, made visible for the treating surgeon on the monitor (not shown) of the computer 98, and stored in the computer 98.

Installed in the computer 98 is image-processing software which serves to determine the coordinates of the geometrical midpoints of all the second projections 119 (points of light) of the second pattern of dots 118 which have arisen out of the ophthalmoscopic projection of the first projections 117 (retinal points of light) of the first pattern of dots 116. The coordinates of the "ideal" points of light 132 which would be generated in the second plane 54 under aberration-free conditions are, as already mentioned, computed on the basis of an aberration-free eye model 130. In accordance with certain embodiments, the actual, for example biometrically measured, ocular length of the eye 12 is used in place of the model ocular length assumed in the eye model. The arrangement of the ideal points of light 132 is the same as the regular two-dimensional arrangement 26 generated by the component-beam-generating device 18 (aperture mask 19) and is magnified merely by a scale of reproduction with respect to the arrangement 26.

The actual ocular length (OL) 198 (see Figures 1, 2, 9 and 10) is measured directly in respect of the patient's eye 12 with a laser-interferometric process. Suitable for this is for example, the aforementioned instrument WaveLight OB 820. This instrument is also suitable to survey, besides the ocular length (OL) 198, also other biometric lengths defined along the first optical axis (e.g. visual axis) of the eye, such as, for instance, the corneal thickness (CT) 192, the anterior-chamber depth (ACD) 194 and the lens thickness (LT) 196 and/or the position of the anterior or posterior side of the lens.

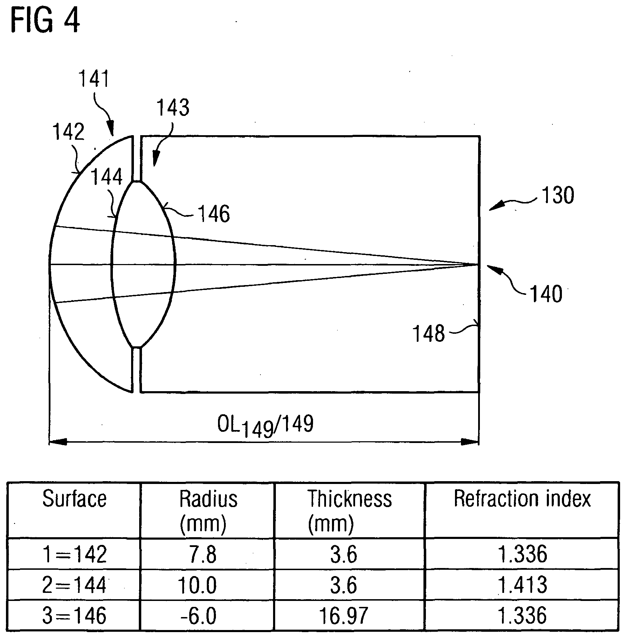

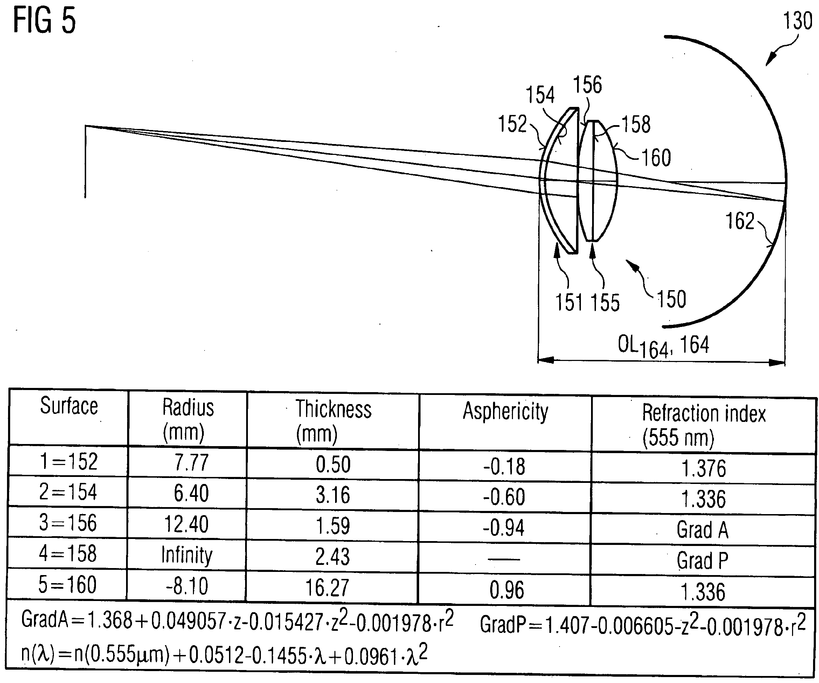

By way of eye model, the Gullstrand eye model 140 represented schematically in Fig. 4 may be employed or the Liou-Brennan eye model 150 represented schematically in Fig. 5. Other eye models may also be employed. For the sake of simplicity, in the following embodiment of the invention will be elucidated on the basis of the Gullstrand eye model. For the other eye models, the use of the actually measured ocular length arises by way of parameter out of the eye model itself.

In the Gullstrand eye model a human eye is approximated by an arrangement shown schematically in Fig. 4 in the upper part. Therein the real optical system of a human eye, consisting of the cornea and the lens which is spaced therefrom, is modelled by a system that consists of a concave-convex front lens 141 with a front face (first surface) 142 and with a back face (second surface) 144, and of a biconcave, rear lens 143, arranged in the direction towards the model retina 148 downstream of the front lens 141, and a back face (third surface) 146. The front face of the rear lens 143 exhibits the same radius of curvature as the back face 144 of the front lens 141 and coincides with the latter. The back face 146 of the rear lens 143 is arranged a defined distance, derived from mean values for actual eyes, upstream of a model retina 148 modelled as a planar face. In other respects, in the Gullstrand eye model 140 the radii of curvature of the first surface 142, of the second surface 144 and of the third surface 146 are set to 7.8 mm, 10.0 mm and -6.0 mm, respectively, as specified in the table in the lower region of Fig. 4. The thickness of the front lens 141 measured along the optical axis of the eye model is set to 3.6 mm, and the refractive index thereof to 1.3358 (rounded 1.336). The thickness of the rear lens 143 is set to 3.6 mm, the refractive index thereof to 1.413, the spacing of the apex of the rear lens surface 146 from the model retina 148 to 16.97 mm, and the refractive index in this region to 1.3358 (rounded 1.336), as likewise specified in the table shown in Fig. 4.

In accordance with certain embodiments, for the model ocular length 149 ($OL_{149}$) defined from the apex of the front face (e.g. the first surface 142) of the front lens 141 to the model retina 148 a value is now taken that corresponds to the actual ocular length (OL) 198 measured in the eye to be examined aberrometrically. Accordingly, deviating from the value specified in the table shown in Fig. 4, the distance from the apex of the back face (i.e. the second surface 146) of the rear lens 143 to the model retina 148 is chosen in such a way that the model ocular length corresponds to the measured, actual ocular length.

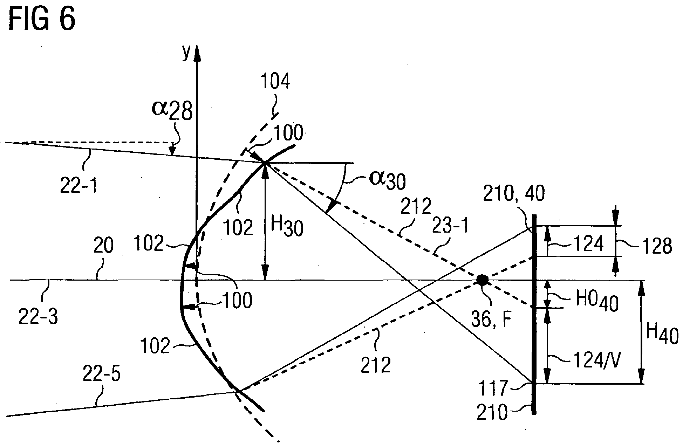

In the Liou-Brennan eye model 150, on the other hand, the optical system of the eye is modelled, in a manner corresponding to reality, by a front concave-convex front lens 151 with a front face (i.e. first surface 152) and with a back face (i.e. second surface 154) which together model a cornea, and by a biconcave rear lens 155, arranged downstream of the front lens 151, with a front face (i.e. third surface 156), with a planar principal plane (i.e. fourth surface 158) and with a back face (i.e. fifth surface 160), and with a model retina 162 in spherical form, as shown in Fig. 5. The optical properties of the optical elements of the Liou-Brennan eye model 150 are summarised in the table in the lower region of Fig. 6. The radii of curvature of the first to fifth surfaces 152, 154, 156, 158 and 160 amount to 7.77 mm, 6.40 mm, 12.40 mm, $\infty$ and -8.10 mm, respectively. The thicknesses of the Liou-Brennan standard model, specified in the table, are defined along the optical axis of the model eye 150 and amount to 0.50 mm (spacing from the first surface 152 to the second surface 154); 3.16 mm (spacing from the second surface 154 to the third surface 156), 1.59 mm (spacing from the third surface 156 to the fourth surface 158), 2.43 mm (spacing from the fourth surface 158 to the fifth surface 160) and 16.27 mm (spacing from the fifth surface 160 to the model retina 162). In other respects, the first, second, third and fifth surfaces 152, 154, 156 and 160 exhibit an asphericity. The refractive index of the region between the first surface 152 and the second surface 154 is set to 1.376; that of the region between the second surface 154 and the third surface 156 is set to 1.336; and that of the region between the fifth surface 160 and the model retina 162 is set to 1.336. The refractive indices of the model of the crystalline lens, i.e. in the region between the third surface 156 and the fourth surface 158 and in the region between the fourth surface 158 and the fifth surface 160, are dependent on the position Z measured along the optical axis and on the spacing R from the optical axis of the model eye 150, as specified by the expressions "degree A" and "degree P" in the table shown in Fig. 5. The dispersion (wavelength dependence) of the refractive indices is likewise modelled in the Liou-Brennan eye model, specifically in accordance with the equation for $n(\lambda)$ specified in the lowest line of the table shown in Fig. 5.

In accordance with certain embodiments, the spacing between the back lens face 160 (fifth surface) and the retina is chosen in such a way that the model ocular length 164 ($OL_{164}$), measured from the apex of the first surface 152 to the model retina 162 along the optical axis, corresponds to the actual ocular length of the eye 12 to be examined aberroscopically.

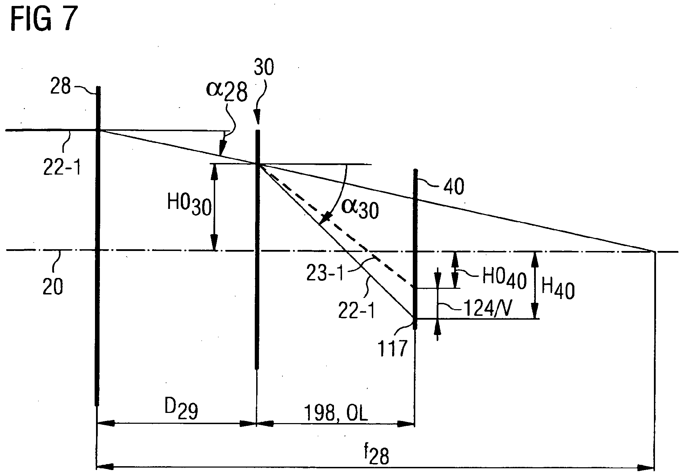

In the following, the process according to certain embodiments for computing the wavefront aberrations 100 of the optical system of the eye is described with reference to Figures 6 to 8. In these embodiments, component beam 22-1 is aberroscopiccally projected by the aberration lens 28 and the optical system 30 of the eye onto the retina 40. As evident in Fig. 7, component beam 22-1 emerging from the component-beam-generating device 18 runs firstly parallel to the first optical axis 20 (i.e. the visual axis) of the eye 12, then passes through the aberration lens 28 and in the process is refracted towards the axis 20 by the angle $\alpha_{28}$. In its further path, component beam 22-1 then passes through the optical system 30 of the eye and in the process is refracted further in the direction towards the optical axis, so that after the passage through the optical system 30 it runs at the angle $\alpha_{30}$ with respect to the axis 20. Light beam 22-1 then impinges on the retina 40 as a projection 117. The point of intersection of light beam 22-1 through the optical system 30 is remote from the first optical axis by the distance $H0_{30}$, see Figures 6 to 8. The point of incidence of the projection 117 of component beam 22-1 on the retina 40 is remote from the first optical axis 20 by the distance $H_{40}$ to be measured; see Figures 6 to 8.

If the optical system 30 of the eye were free from imaging errors (aberrations), component beam 22-1 would be refracted on the optical system 30 at a different (in the example of Figures 6 and 7, smaller) angle towards the first optical axis 20, and take the path 23-1 (along the dashed line in Figures 6 and 7) and impinge on the retina at a distance $H0_{40}$ from the first optical axis 20. The offset arising on the retina 40 between the point of incidence of component beam 22-1 impinging on the aberration-burdened optical system 30 of the eye (spacing from the optical axis: $H_{40}$) and the point of incidence of the refracted component beam 23-1 on the ideal, aberration-free system (spacing from the optical axis: $H0_{40}$) constitutes the offset to be measured.

The offset in the second plane 54 arranged outside the eye 12, i.e. in the image plane of the recording device 60 (CCD camera 62), is measured, as elucidated above with regard to Fig. 3. The magnification arising in the course of the ophthalmoscopic projection of the retina 40 into the second plane 54 (projection magnification) is denoted in Fig. 3 and in the following equations by the symbol V. The offset 124 shown in Figures 6 and 7 on the retina 40 between the points of incidence of component beam 22-1 projected in aberration-burdened manner (projection 117) and component beam 23-1 projected in aberration-free manner is expressed formalistically in Figures 6 and 7 as the offset 124, shown in Fig. 3 and measurable in the second plane 54 (i.e. in the image plane of the recording device 60), between the second projection 119 of component beam 22-1, projected onto the retina in aberration-burdened manner, and the projection 132 of component beam 23-1, projected onto the retina in aberration-free manner (see Figures 6 and 7), divided by the projection magnification V of the ophthalmoscopic projection. Conversely, in the section, drawn on a magnified scale in Fig. 3 in the lower region, of a second pattern of dots 118 generated in the second plane 54 the offset 124 between the projection 132 of component beam 23-1 which is projected in aberration-free manner and the second projection 119 of component beam 22-1 which is projected in aberration-burdened manner is expressed by the difference in the spacing of the points of incidence on the retina 40, as shown in Figures 6 and 7, i.e. spacing from point $H_{40}$ to point $H0_{40}$ multiplied by the projection magnification V of the ophthalmoscopic projection.

The wavefront aberration of the eye can be determined from the change in the angle $\alpha$ of each component beam 22-1, ..., 22-5 upon passing through the optical system 30 of the eye. As already mentioned, by virtue of the aberroscopic projection a first pattern of dots 116 is generated on the retina 40, which in the aberration-free case would appear undistorted (i.e. without offset) with respect to the regular two-dimensional arrangement 26, generated by the component-beam-generating device 18, of the component beams on the retina 40. In the emmetropic case, from the component beams 22-1, ..., 22-5 a sharp image point should arise on the retina by virtue of the aberroscopic projection through the optical system 30 of the eye. By virtue of the insertion of the aberroscope lens 28, however, for each component beam 22-1,..., 22-5 a defined spacing $H_{40}$ of the projection (of the point of light) from the first optical axis 20 (i.e. visual axis) of the eye is generated on the retina. The spacing $H_{40}$ can be computed from the following equation (1):

$$HO_{40} \;=\; \frac{HO_{30}}{f_{28} - D_{29}} \times OL \qquad\qquad (1)$$

In equation (1)

$H0_{40}$    stands for the spacing, caused by the aberroscope lens 28 on the retina 40, of the point of incidence 117 from the first optical axis 20,

$H0_{30}$    stands for the spacing of the point of intersection of the corresponding component beam, for example 22-1, through the optical system 30 of the eye from the first optical axis 20,

$f_{28}$    stands for the focal length of the aberroscope lens 28,

$D_{29}$    stands for the spacing between the aberroscope lens and the anterior face of the eye, and

OL    stands for the ocular length.

Prior to the development of the instruments that have been developed for the purpose of measuring lengths and depths, inclusive of the ocular length, the ocular length OL was not measurable. Therefore in equation (1) it was estimated or assumed approximately by the eye-specific, spherical equivalent $D_r$ measured previously:

$$OL \;=\; OL_{149} + d(OL) \;=\; \frac{1000 \times n}{(D_{140} + D_r)} \qquad\qquad (2)$$

Equation (2) holds only for the simplified Gullstrand model 140 of the eye. In equation (2)

$OL_{149}$ stands for the ocular length of the simplified Gullstrand eye model, which is set to 22.36 mm,
$D_{140}$ stands for the refractive power of the optical system of the Gullstrand eye model 140, and
n stands for the refractive index of the material of the optical system of the Gullstrand eye model 140.

The actual spacing, generated by the aberration-burdened projection through the optical system 30 of the eye, of the projection 117 of a component beam 22-1 on the retina 40 can be computed from the spacings $H_{54}$, measured in the second plane 54, of the second projections 119 from the second optical axis 52, in accordance with:

$$H_{40} = \frac{H_{54}}{V} = \frac{HPix}{V} \times \frac{CCD}{CCDPix} \qquad (3)$$

In equation (3)

$H_{40}$ stands for the spacing of the first projection 117 of component beam 22-1, projected in aberration-burdened manner, from the first optical axis 20 (e.g. visual axis) on the retina 40 of the eye,
$H_{54}$ stands for the spacing of the projection 119 in the second plane 54, i.e. in the image plane of the ophthalmoscopic projection in the recording device 60, from the second optical axis 52, and
V stands for the magnification factor of the ophthalmoscopic projection of the retina in the second plane 54,
HPix stands for the spacing of the second projection 119 in the second plane 54 from the second optical axis 52 (measured in pixel units of the CCD camera 62),
CCD stands for the size of the CCD sensor of the camera 62 (expressed in mm), and
CCDPix stands for the resolution of the CCD sensor of the camera 62 (expressed in pixel units).

From equations (1) and (3) the coordinates are obtained of the point of intersection of a component beam in the optical system 30 of the eye ($H_{30}$ predetermined by the aberroscope lens 28), the coordinates of the point of incidence of component beam 23-1 on the retina 40 in the aberration-free case ($H0_{40}$: spacing from the optical axis), and the coordinates of the actual point of incidence 117 of component beam 22-1 on the retina 40 ($H_{40}$: spacing from the optical axis), as determined from the ophthalmoscopic projection into the second plane 54; in this regard see Figures 3 and 7.
From the data $H0_{30}$, $H0_{40}$ and $H_{40}$ the angles of the component beams with respect to the first optical axis 20 in the plane of the optical system 30 (of the cornea 32) can be computed as follows or read off from Fig. 7:

$$\tan \alpha_{28} = \frac{H0_{30}}{f_{28} - D_{29}} + \frac{H0_{30}}{OL} = H0_{30} \times \frac{f_{28} - D_{29} + OL}{(f_{28} - D_{29}) \times OL} \qquad (4)$$

$$\tan \alpha_{30} = \frac{H0_{30}}{f_{28} - D_{29}} + \frac{H0_{30} + H_{40} - H0_{40}}{OL} \qquad (5)$$

In equations (4) and (5)

$\alpha_{28}$ stands for the angle that arises for component beam 22-1 by virtue of the refraction on the inserted aberroscope lens 28,
$\alpha_{30}$ stands for the angle that arises by virtue of the refraction on the aberroscope lens 28 and the refraction on the optical system 30 of the eye.

By inserting equation (1), equation (5) is simplified as follows:

$$\tan \alpha_{30} = \frac{H0_{30} + H_{40}}{OL} \qquad (6)$$

For a highly myopic eye also no aberration lens 28 may be employed. For this case, in the above equations

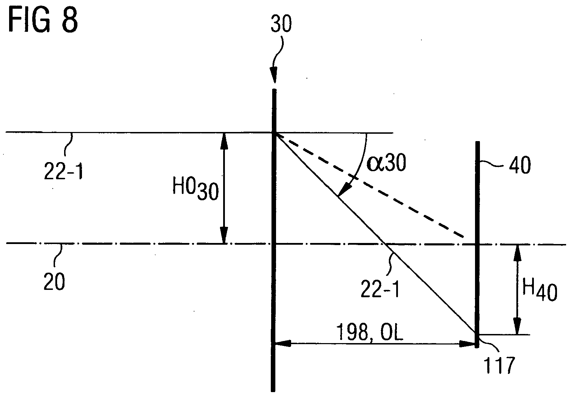

(1) to (5) $\alpha_{28} = 0$ holds, and the beam path shown in Fig. 7 is simplified into the beam path shown in Fig. 8. Equation (6) also holds for this case.

The angle of the component beams, which arises solely by virtue of the presence of the aberrations of the optical system 30 of the eye, is obtained for the two cases of the emmetropic eye (Fig. 7) and of the highly myopic eye (Fig. 8) by forming the difference (i.e. by subtraction) of the angles $\alpha_{30}$ and $\alpha_{28}$. The increase dW in the wavefront aberration, i.e. the change in the wavefront aberration with the radial spacing of the point of intersection through the optical system 30 from the first optical axis 20, is then the tangent of the difference angle:

$$dW = \tan\left(\arctan\left(\tan\alpha_{30}\right) - \arctan\left(\tan\alpha_{23}\right)\right) \qquad (7)$$

As can be seen by inserting equations (4) and (6) into the expression on the right-hand side in equation (7), the computation of dW uses the focal length of the aberroscope lens 28 ($f_{28}$), the spacing of the aberroscope lens 28 from the anterior face of the eye ($D_{29}$), the ocular length OL, the coordinates of the points of intersection ($x_k$, $y_k$) of the component beam through the optical system 30 of the eye, and the coordinates ($H_{40}$), determined from the aberroscopic measurement, of the actual points of incidence 117 (points of light) of the component beams on the retina 40. The quantities for the computation of dW are either predetermined by the optical system of the aberroscope, i.e. the apparatus 10, or can be computed from the above equations (7), (6) and (4).

In the following, the representation of the wavefront aberrations according to the decomposition due to Zernike, i.e. the representation of the wavefront aberration 100 generated by the optical system 30 of the eye as a sum of Zernike polynomials normalised to a unit circle and weighted with corresponding Zernike polynomial coefficients, will be elucidated with reference to Tables 1 to 6.

As a result of the evaluation of the ophthalmoscopically projected, second pattern of dots 118 recorded by the recording device 60, i.e. from the measurement, effected for each component beam taken into account, of the offset 124 of component beam 22-1 which is projected in aberration-burdened manner with respect to component beam 23-1 which is projected without aberrations, a set of m coordinate pairs ($x_k$, $y_k$) is obtained with k = 1, ... , m for the points of incidence of the component beams 22-1, ... , 22-m which are projected in aberration-burdened manner through the optical system of the eye. In this case the coordinates ($x_k$, $y_k$) of the points of incidence 119 are referred to the second optical axis 52, i.e. they are relative coordinates. From these coordinates the deviation of the reconstructed, real or actual wavefront 102 with respect to an ideal, i.e. planar, wavefront 104 can now be computed as wavefront aberrations 100 shown in Fig. 6.

The wavefront aberration may be mathematically represented with respect to the coordinates ($x_k$, $y_k$) in the image plane (second plane 54), a suitable descriptive form is chosen. The Zernike polynomials for a sum representation of the wavefront aberrations may be used. According to this, the following formulation is made:

$$W(x_k, y_k) \;=\; \sum_{i=0}^{N} C_i Z_i(x_k, y_k) \qquad (8)$$

In equation (8)

$W(x_k, y_k)$      stands for the wavefront aberration 100, a two-dimensional function defined at the points ($x_k$, $y_k$),
$Z_i(x_k, y_k)$      stands for the i-th Zernike polynomial defined at the point ($x_k$, $y_k$),
$C_i$      stands for a ($C_i$) of the coefficients ($C_1$, ... , $C_N$) to be determined of the respective Zernike polynomial $Z_i$,
$N$      stands for the number of Zernike polynomials drawn upon for the sum decomposition, and
$i$      stands for a summation index.

The Zernike polynomials are defined by equations (19) and (20), specified further below, in polar coordinates (r, $\phi$) and are computed algebraically and listed in polar coordinates in Table 1 (see the Appendix with Tables 1 to 6) in accordance with equations (19) and (20).

According to the definition of the Zernike polynomials , N assumes defined values as specified in the final column of Table 3. Also, only decompositions with complete polynomial orders n and, at the same time, also only the use of even orders, for example n = 2, 4, 6, 8, ..., are meaningful. Thus, for example, including in the decomposition, in addition to N = 27 polynomials of the n = 6th order, also a 28th or 29th polynomial affords no increase in knowledge.

However, as elucidated above with reference to Figures 6 to 8, from the pattern 116 of the points of light (first projections) 117 projected onto the retina 40 only the increases dW in the wavefront aberration at the points ($x_k$, $y_k$) are obtained, i.e. the change in the wavefront aberration with the coordinates in the plane of projection, i.e. the first partial

derivatives $\left(\dfrac{\partial W}{\partial x}\right)$ and $\left(\dfrac{\partial W}{\partial y}\right)$ of the two-dimensional function W with respect to the coordinates x and y in the plane of projection (second plane 54):

$$\frac{\partial W\,(x_k,y_k)}{\partial x} = \sum_{i=0}^{N}\left[C_{i,x}\,\frac{\partial Z_i(x_k,y_k)}{\partial x}\right] \qquad (9)$$

and

$$\frac{\partial W\,(x_k,y_k)}{\partial y} = \sum_{i=0}^{N}\left[C_{i,y}\,\frac{\partial Z_i(x_k,y_k)}{\partial y}\right] \qquad (10)$$

This means that from the determination of the offsets of the points of incidence 117 in the Zernike decomposition for the total of m coordinate pairs $(x_k,\,y_k)$ two equation systems result:

$$\mathbf{W}_x = \mathbf{CZ}_x \qquad \mathbf{W}_y = \mathbf{CZ}_y$$

with

$$\mathbf{W}_x = \begin{pmatrix} \frac{\partial W(x_1,y_1)}{\partial x} \\ \vdots \\ \frac{\partial W(x_m,y_m)}{\partial x} \end{pmatrix} \quad \mathbf{W}_y = \begin{pmatrix} \frac{\partial W(x_1,y_1)}{\partial y} \\ \vdots \\ \frac{\partial W(x_m,y_m)}{\partial y} \end{pmatrix} \quad \mathbf{C} = \begin{pmatrix} C_1 \\ \vdots \\ C_n \end{pmatrix} \qquad (11)$$

$$\mathbf{Z}_x = \begin{pmatrix} \frac{\partial Z_1(x_1,y_1)}{\partial x} & \cdots & \frac{\partial Z_n(x_1,y_1)}{\partial x} \\ \vdots & \vdots & \vdots \\ \frac{\partial Z_1(x_m,y_m)}{\partial x} & \cdots & \frac{\partial Z_n(x_m,y_m)}{\partial x} \end{pmatrix} \quad \mathbf{Z}_y = \begin{pmatrix} \frac{\partial Z_1(x_1,y_1)}{\partial y} & \cdots & \frac{\partial Z_n(x_1,y_1)}{\partial y} \\ \vdots & \vdots & \vdots \\ \frac{\partial Z_1(x_m,y_m)}{\partial y} & \cdots & \frac{\partial Z_n(x_m,y_m)}{\partial y} \end{pmatrix}$$

A standard equation method is unsuitable for the solution of this equation system. A numerical stability in the numerical solution of these equation systems is obtained by using a solution method such as singular value decomposition (SVD).

The solution of the equation systems is only calculable when at least as many measuring-points $(x_k,\,y_k)$ or interpolation nodes as Zernike polynomials (in this case the unknowns) are present:

$$m \geq N \qquad \text{or better:} \qquad m \geq 2N \qquad (12)$$

As already mentioned, in equation (12) m stands for the number of measuring-points, and N for the number of Zernike polynomials drawn upon in the sum decomposition.

Conversely, equation (12) means that the order of the Zernike polynomials that is capable of being taken into account depends on the number of points of light 117 that are capable of being evaluated in the image generated aberroscopically on the retina.

For example, given the apparatus 10 shown in Fig. 2 with the component-beam-generating device 18 (aperture mask 19) described above, see the left-hand part of Fig. 3, and a pupil with 6 mm diameter, there are theoretically 68 measuring-points or coordinate pairs, so that a decomposition with Zernike polynomials can be computed as far as the 6th order.

It is to be noted, furthermore, that the Zernike polynomials are only defined on a unit circle. So the coordinates of the measuring-points $(x_k,\,y_k)$ have to be normalised prior to the computation:

$$X_k = \frac{x_k}{R}, \quad Y_k = \frac{y_k}{R} \qquad (13)$$

In equation (13), R stands for the radius of the Zernike circle, i.e. the circle on which the computation is effected, and $X_k$ or $Y_k$ stands for the coordinates normalised to the Zernike circle.

The radius R of the Zernike circle corresponds on the eye to the radius of the pupil, and it should firstly be capable of being chosen freely. The radius may be adjusted, especially for use with the laser, in accordance with the optical zone being aimed at for the aberrometric characterisation. All the points of light that lie outside the width of the pupil corresponding to the Zernike circle radius R do not typically enter into the computation. So for the purpose of solving the equation system only measuring-points are permitted to be drawn upon for which the following holds:

$$\sqrt{x_k^2 + y_k^2} \;\leq\; R \qquad\qquad (14)$$

The resultant vector C introduced into equation (11), which includes the coefficients $C_1$, ..., $C_n$, also holds only for the Zernike circle (unit circle). In order now to determine the actual wavefront, the normalised wavefront aberration has to be linearly scaled with the radius of the pupil. Lastly, for the representation of the results it is still sensible to specify the Zernike coefficients $C_n$ in micrometres. In the example elucidated above on the basis of equations (1) to (7), the Zernike coefficients are obtained in millimetres.

From the Zernike coefficients, the spherical and the cylindrical refractions of the eye being examined can be determined directly:

$$cyl = -\frac{4}{R^2}\sqrt{C_3^2 + C_5^2} \qquad\qquad (15)$$

$$sph = -\frac{4}{R^2}C_4 - \frac{1}{2}cyl \qquad\qquad (16)$$

$$\phi = \frac{1}{2}\arctan\!\left(\frac{C_3}{C_5}\right) + \varphi \qquad\qquad (17)$$

with: $\varphi = 90°$ if $C_5 < 0$, otherwise: $\varphi = 180°$ if $C_3 < 0$, otherwise $\varphi = 0°$ (18).

The conditions stated in equation (18) are dependent on the range of values of the arctangent in the compiler that is being used for the computer program that has been developed for solving the equation systems. For the compilers that are used in the present case it holds that: $-90° \leq \arctan \leq +90°$.

For the purpose of completing the representation of the sum decomposition introduced above in equation (8) by means of Zernike polynomials, in the following the use of the Zernike polynomials with regard to the ocular length (OL) is presented.

The Zernike polynomials are originally defined for cylindrical coordinates (r, $\phi$) introduced in the plane of projection with respect to the point of intersection of the optical axis as follows:

$$Z(r,\phi) = \begin{cases} \sqrt{2(N+1)}\,R_n^k(r)\sin\!\big(|N-2m|\phi\big) & if \quad N-2m > 0 \\ \sqrt{2(N+1)}\,R_n^k(r)\cos\!\big(|N-2m|\phi\big) & if \quad N-2m < 0 \\ \sqrt{N+1}\,R_n^k(r) & if \quad N-2m = 0 \end{cases} \qquad (19)$$

In equation (19) the following holds for the quantities $R_n^k(r)$ :

$$R_n^k(r) = \sum_{i=0}^{k} \frac{(-1)^i (n-1)!}{i!(k-i)!(n-k-i)!},\qquad (20)$$

with:

$$k = \begin{cases} m & N-2m \geq 0 \\ N-m & N-2m < 0. \end{cases}$$

In this connection it holds for the variables that:

$$0 \leq N \leq \infty$$

and

$$0 \leq m \leq n.$$

In Table 1 the Zernike polynomials, expressed in polar coordinates $(r, \phi)$, are compiled up to and including the 8th order (n = 8), that is to say, the first 45 polynomials (N = 0, 1, 2, ..., 44), as obtained by algebraic evaluating of equations (19) and (20).

In Table 1 (fourth column) and in Table 4 (second column) the radical from equation (19) in front of the actual polynomial is denoted by the symbol F. Table 4 lists the Zernike polynomials expressed in Cartesian coordinates (x, y).

For the evaluation of the second pattern of dots 118 recorded by the recording device 60 a conversion of the Zernike polynomials into the Cartesian coordinates x and y defined in the plane of projection (second plane 54) becomes necessary. The conversion of polar coordinates $(r, \phi)$ (as utilised in Table 1) into Cartesian coordinates (x, y) is effected with the aid of the following known rules:

$$y = r\sin\phi, \quad x = r\cos\phi \quad \text{and} \quad r^2 = x^2 + y^2 \qquad (21)$$

The complicated trigonometric terms arising in the course of the conversion of the in Table 1, by the exponentiation of the polar coordinates r, can be simplified by using the summation formulae for trigonometric functions specified in Table 2. The powers of the trigonometric functions arising after the simplification can then be converted into Cartesian coordinates, and in this way the Zernike polynomials listed in Table 4 can be computed algebraically, expressed in Cartesian coordinates (x, y).

The results of the conversions of the first 45 Zernike polynomials from Table 1 into the Zernike polynomials expressed in Cartesian coordinates are summarised in Table 4.

Listed in Tables 5 and 6, lastly, are the first partial derivatives of the Zernike polynomials with respect to the coordinates x and y (i.e. the functions $\partial W/\partial x$ and $\partial W/\partial y$), which have been computed algebraically from the Zernike polynomials specified in Table 4.

It is the partial derivatives of the Zernike polynomials, compiled in Tables 5 and 6, that are ultimately needed for solving the equation systems (11), taking as a basis the measured offsets of the individual points of light and the subsequent determination of the wavefront aberrations.

Since the mathematical foundations for the reconstruction of the improved wavefront aberrations have been described, in the following it will be now be described with reference to Figures 9 to 13 how, on the basis of the actual wavefront 102 (see Fig. 6) acquired therefrom and generated by the optical system of the eye, and also, where appropriate, on the basis of further data measured in respect of the eye,

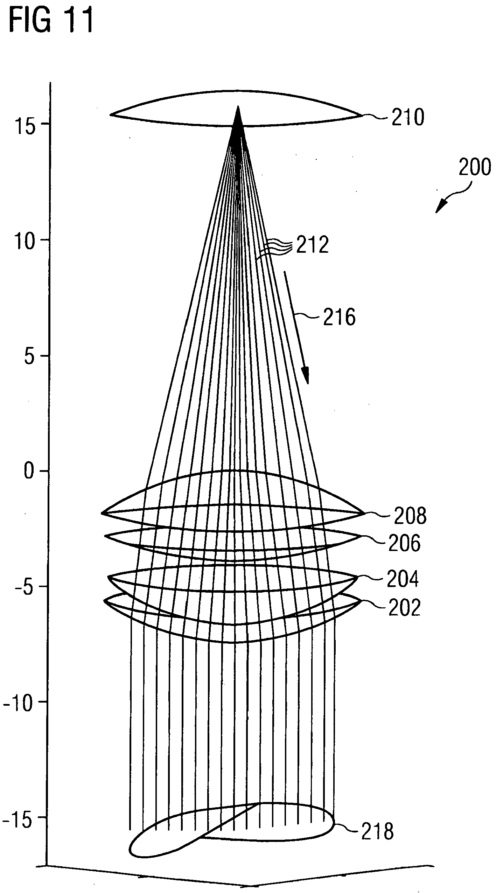

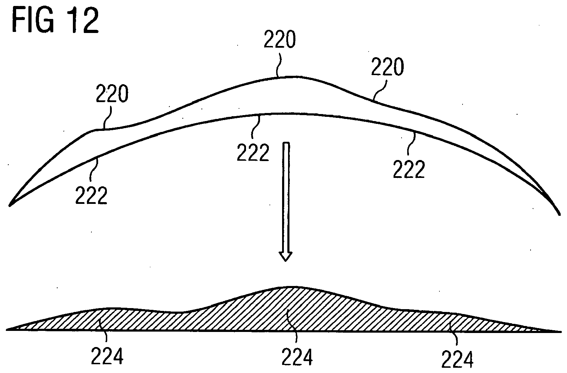

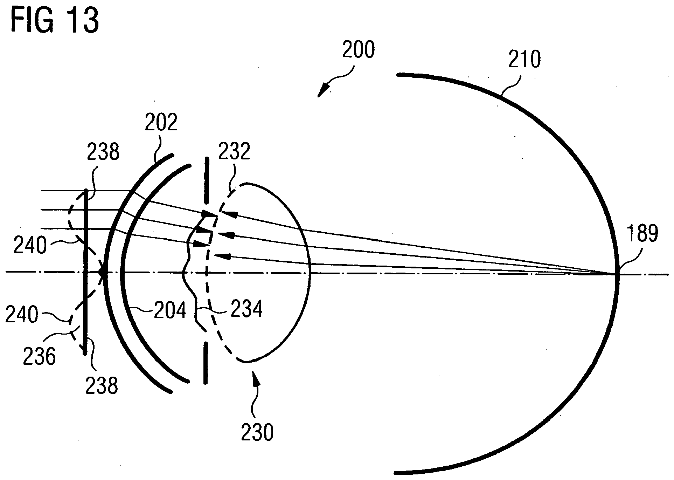

- an improved, eye-specific eye model 200 (see Figures 9, 10 and 11),
- on the basis of the improved, eye-specific eye model 200 (see Fig. 11), an improved ablation profile 224 for a laser-surgical refraction correction of the pre-operative anterior corneal surface 220 of the eye (see Fig. 12), and
- on the basis of the improved, eye-specific eye model 200 (see Fig. 11), an improved design or an improved front lens face 232, for an intraocular lens 230 (IOL) to be surgically inserted into the eye for the purpose of correcting imaging errors of the optical system of the pre-operative eye (see Fig. 13) are computed.

In certain embodiments, further data may be obtained, e.g., the topography of the anterior corneal surface 174 and of the posterior corneal surface 176 as well as biometric data pertaining to the eye, inclusive of the corneal thickness (CT) 192, the anterior-chamber depth (ACD) 194, the lens thickness (LT) 196 and the ocular length (OL) 198, see in each instance Fig. 9.

Fig. 9 shows the data to be measured in a schematic eye model 200. An axially symmetrical eye model is chosen as the starting point for the computations. For this purpose the Gullstrand eye model 140 (see Fig. 4) or the Liou-Brennan eye model 150 (see Fig. 5) can be used, for example. The choice of an axially symmetrical eye model is appropriate at this stage of the creation of the eye model, because the exact position of the corneal surface generally cannot be determined by measurements. Also further intraocular structures - such as the shape, locus dependence and locus distribution of the refractive index of the lens - likewise generally cannot be measured directly. Nevertheless, these can be computed numerically on the basis of wavefront measurements of the entire eye. For this purpose, in a first step, mean values for the data pertaining to the lens (front and rear surfaces, refractive index and distribution thereof) are taken from the literature and introduced into the axially symmetrical eye model chosen as a starting point.

The basis for optical computations using an eye model is the possibility of tracking beams of light computationally using ray tracing. In the course of the ray tracing, the optical path of beams of light that pass through the eye is computed utilising Snell's known law of refraction. The dependence of the refractive index on the wavelength is taken into account in this case through a suitable choice of the refractive indices for the various optical media of the eye model.

In order that the simulated wavefront for the entire individualised, improved eye-specific eye model to be created now becomes identical with the actual wavefront 102 (see Figures 6 and 9) measured in respect of the eye, the shape of the lens 180 is adapted by an iterative optimisation algorithm. In this algorithm, either the front lens face 182 or the back lens face 184 of the lens 180 or, in the case of a weighted optimisation, both surfaces 182 and 184, is/are adapted.

The optimisation algorithm has to be performed in an iterative manner, because the points of intersection of the tracked beams of light through the surfaces to be optimised 182 and 184 are required in order to start the algorithm, even though the surfaces 182 and 184 are not yet known. For this reason, by way of initial formulation for the iteration the lens was modelled with mean-value data pertaining to an eye model taken from the literature. The lens surface, which in this process is not yet optimised, is still the lens shape according to the bibliographical references for the underlying initial eye model.

The objective of the intraocular optimisation is to adapt a refracting surface in such a way that a defined wavefront upstream of the surface to be optimised can be transformed, by virtue of the refraction on the surface, into a wavefront defined downstream of the surface. In a manner analogous to the principle used in a Hartmann-Shack wavefront sensor it will be assumed that the measured wavefront above the entrance pupil resembles the respective wavefront that leaves the eye, starting from the ideal focal point 189 on the model retina 210 in Fig. 10 (188 in Fig. 9), see Fig. 10. In the case shown in Fig. 10 of an optimisation of the front lens face 232 (182 in Fig. 9), the wavefront 102 measured upstream of the eye is tracked through the anterior corneal surface 202 and the posterior corneal surface 204 "into the eye", in order to compute the intraocular wavefront 234 immediately upstream of the front lens face 232 (see in Fig. 10 the left-hand side with regard to the front lens face 232). On the other hand, a ray tracing from the ideal focal point 189 on the model retina 210 is performed "out of the eye" through the back lens face 237 of the lens, in order to compute the point of intersection of the beams of light through the front lens face 232 (see in Fig. 10 the right-hand side with respect to the front lens face 232). By utilising Snell's law of refraction, the face inclinations of the front lens face 232 can then be computed. Lastly, the shape of the front lens face 232 can be determined by a function describing the shape being derived and being adapted to the computed face inclinations. As already mentioned, the entire process has to be performed iteratively. The entire process is applied both to the ray tracing performed through the cornea "into the eye" and to the ray tracing carried out from the retina "out of the eye". It is possible that the solution of the optimisation algorithm is non-standard, because an identical wavefront error due to change can also be brought about by other surfaces of the eye model. However, this effect will, as expected, only show differences for a so-called off-axis (i.e. not running parallel to the optical axis) analysis of the eye model, whereas for the foveal visual faculty mutually consistent results are expected for the two ray tracings.

As a result of the two iteratively performed optimisations indicated in Fig. 10, after the adaptation of the front lens face 232 a completely individualised improved eye model 200 for a patient is obtained. An individualised improved eye model 200 obtained in this way is shown in exemplary manner in Fig. 11. In Fig. 11 a bundle of light rays 212, which emanate from the model retina 210 in an initial direction 216, is tracked by the entire optical system of the eye model 200 - constituted by the adapted back lens face 208, the adapted front lens face 206 obtained, the posterior corneal surface 204 and the anterior corneal surface 202 - right up to the eye with a ray-tracing program, and the wavefront 218 generated upstream of the eye by the bundle of rays emerging from the eye is shown.

Since an improved, individualised eye-specific eye model 200 for the eye of a patient has been created, this model can be taken for various applications of treatment planning, inclusive of the planning or advance computation of an optimised ablation profile for the anterior surface of the cornea (see Fig. 12), of the computation of the shape of an intraocular lens (IOL, see Fig. 13) to be inserted into the eye, or of a combination of these two forms of treatment planning. The individualised eye-specific eye model 200 has been created in such a way that the wavefront 218 generated by the eye model 200 coincides with the wavefront 102 (see Fig. 6) measured in respect of the eye by means of the aberrometer 10, so the improved eye model 200 automatically contains the imaging errors or aberrations, inclusive of the higher-order aberrations of the eye of the patient. The aforementioned optimisation algorithms may be performed iteratively and are based on the eye model that uses the actual ocular length as the model ocular length. Also, the actual ocular length may be used in the subsequent adaptations and optimisations.

In order to compute a laser ablation profile for the anterior corneal surface, the improved, individualised eye model 200 which has been created (see Fig. 11) is now utilised in order to compute an optimal shape of the anterior corneal surface with the desired optical quality. This computation is performed in two stages as follows:

stage 1: 3-dimensional ray tracing, starting from the model retina 210 as far as the cornea, and

stage 2: iterative computation of the ideal shape 222 of the anterior corneal surface (see Fig. 12, upper region).

The computation in stage 2 also has to be performed iteratively, because the points of intersection of the beams through the newly to be computed face 222 are not known initially.

An improved ablation profile 224 (see Fig. 12, lower region) is then obtained from the difference or discrepancy between the topography of the anterior corneal surface 220 of the improved, individualised eye model 200 and the computed ideal shape 222 of the anterior corneal surface (see Fig. 12). The improved ablation profiles 224 obtained as described above enable a more accurate characterisation of the defective vision, inclusive of the higher-order aberrations.

Lastly, the process for computing an improved design of an intraocular lens (IOL) 230 to be inserted into the eye will be described in the following with reference to Fig. 13.

In the course of the computation of the improved design for an intraocular lens 230 it is not the primary objective, but a possible future objective, to correct higher-order aberrations; this is rather the objective for the optimisation of the improved ablation profile 224 for the refraction correction in respect of the anterior corneal surface. Therefore, for purposes of the optimisation of the IOL 230, eye models with lower degrees or demands made of the individualisation can also be utilised.

In contrast to the optimisation of the anterior corneal surface, as described on the basis of Figures 10 and 11, an intraocular surface can be optimised in a manner similar to that in the case of the individualisation, described with regard to Fig. 10, of the eye model 200, see now Fig. 13 in this regard. Firstly, for the purpose of generating an intended wavefront of the overall eye, whereby the intended wavefront may be a flat target wavefront 238 or a target wavefront 240 with multifocal properties (i.e. burdened also with higher-order aberrations), the beams of light going "out of the eye" from the ideal projection point 189 on the model retina 210 are tracked through the anterior corneal surface 202 and the posterior corneal surface 204 of the eye model 200, whereby in this case an intraocular wavefront 234 is computed immediately upstream of the front lens face 232 to be optimised (see Fig. 13).

The actual optimisation of the front lens face then takes place, once again iteratively, by ray tracing, again starting from the ideal projection point 189 on the model retina 210. In the course of this ray tracing, the front face 232 of the intraocular lens 230 to be designed is now adapted, in order ultimately to generate, as well as possible, the wavefront 234 computed upstream of the improved front lens face 232.

## Table 1: The first 45 Zernike polynomials

| N | n | m | F | Polynomials in polar coordinates | Optical description |
|---|---|---|---|---|---|
| 0 | 0 | 0 | 1 | $1$ | constant shift |
| 1 | 1 | 0 | 2 | $r\sin\varphi$ | inclination in y-direction |
| 2 | 1 | 1 | 2 | $r\cos\varphi$ | inclination in x-direction |
| 3 | 2 | 0 | $\sqrt6$ | $r^2\sin2\varphi$ | astigmatism ±45° |
| 4 | 2 | 1 | $\sqrt3$ | $2r^2-1$ | focus shift |
| 5 | 2 | 2 | $\sqrt6$ | $r^2\cos2\varphi$ | astigmatism 0°/90° |
| 6 | 3 | 0 | $\sqrt8$ | $r^3\sin3\varphi$ | 3-fold along y |
| 7 | 3 | 1 | $\sqrt8$ | $(3r^2-2)r\sin\varphi$ | coma along y |
| 8 | 3 | 2 | $\sqrt8$ | $(3r^2-2)r\cos\varphi$ | coma along x |
| 9 | 3 | 3 | $\sqrt8$ | $r^3\cos3\varphi$ | 3-fold along x |
| 10 | 4 | 0 | $\sqrt{10}$ | $r^4\sin4\varphi$ | 4-fold along y |
| 11 | 4 | 1 | $\sqrt{10}$ | $(4r^2-3)r^2\sin2\varphi$ | astigmatism $r^4$ ±45° |
| 12 | 4 | 2 | $\sqrt5$ | $6r^4-6r^2+1$ | spherical aberration |
| 13 | 4 | 3 | $\sqrt{10}$ | $(4r^2-3)r^2\cos2\varphi$ | astigmatism $r^4$ 0°/90° |
| 14 | 4 | 4 | $\sqrt{10}$ | $r^4\cos4\varphi$ | 4-fold along x |
| 15 | 5 | 0 | $\sqrt{12}$ | $r^5\sin5\varphi$ | 5-fold along y |
| 16 | 5 | 1 | $\sqrt{12}$ | $(5r^2-4)r^3\sin3\varphi$ | 3-fold $r^5$ along y |
| 17 | 5 | 2 | $\sqrt{12}$ | $(10r^4-12r^2+3)r\sin\varphi$ | coma $r^5$ along y |
| 18 | 5 | 3 | $\sqrt{12}$ | $(10r^4-12r^2+3)r\cos\varphi$ | coma $r^5$ along x |
| 19 | 5 | 4 | $\sqrt{12}$ | $(5r^2-4)r^3\cos3\varphi$ | 3-fold $r^5$ along x |
| 20 | 5 | 5 | $\sqrt{12}$ | $r^5\cos5\varphi$ | 5-fold along x |
| 21 | 6 | 0 | $\sqrt{14}$ | $r^6\sin6\varphi$ | 6-fold along y |
| 22 | 6 | 1 | $\sqrt{14}$ | $(6r^2-5)r^4\sin4\varphi$ | 4-fold $r^6$ along y |
| 23 | 6 | 2 | $\sqrt{14}$ | $(15r^4-20r^2+6)r^2\sin2\varphi$ | astigmatism $r^6$ ±45° |
| 24 | 6 | 3 | $\sqrt7$ | $20r^6-30r^4+12r^2-1$ | spherical aberration $r^6$ |
| 25 | 6 | 4 | $\sqrt{14}$ | $(15r^4-20r^2+6)r^2\cos2\varphi$ | astigmatism $r^6$ 0°/90° |
| 26 | 6 | 5 | $\sqrt{14}$ | $(6r^2-5)r^4\cos4\varphi$ | 4-fold $r^6$ along x |
| 27 | 6 | 6 | $\sqrt{14}$ | $r^6\cos6\varphi$ | 6-fold along x |
| 28 | 7 | 0 | 4 | $r^7\sin7\varphi$ | 7-fold along y |
| 29 | 7 | 1 | 4 | $(7r^2-6)r^5\sin5\varphi$ | 5-fold $r^7$ along y |
| 30 | 7 | 2 | 4 | $(21r^4-30r^2+10)r^3\sin3\varphi$ | 3-fold $r^7$ along y |
| 31 | 7 | 3 | 4 | $(35r^6-60r^4+30r^2-4)r\sin\varphi$ | coma $r^7$ along y |
| 32 | 7 | 4 | 4 | $(35r^6-60r^4+30r^2-4)r\cos\varphi$ | coma $r^7$ along x |
| 33 | 7 | 5 | 4 | $(21r^4-30r^2+10)r^3\cos3\varphi$ | 3-fold $r^7$ along x |
| 34 | 7 | 6 | 4 | $(7r^2-6)r^5\cos5\varphi$ | 5-fold $r^7$ along x |
| 35 | 7 | 7 | 4 | $r^7\cos7\varphi$ | 7-fold along x |
| 36 | 8 | 0 | $\sqrt{18}$ | $r^8\sin8\varphi$ | 8-fold along y |
| 37 | 8 | 1 | $\sqrt{18}$ | $(8r^2-7)r^6\sin6\varphi$ | 6-fold $r^8$ along y |
| 38 | 8 | 2 | $\sqrt{18}$ | $(28r^4-42r^2+15)r^4\sin4\varphi$ | 4-fold $r^8$ along y |
| 39 | 8 | 3 | $\sqrt{18}$ | $(56r^6-105r^4+60r^2-10)r^2\sin2\varphi$ | astigmatism $r^8$ ±45° |
| 40 | 8 | 4 | 3 | $70r^8-140r^6+90r^4-20r^2+1$ | spherical aberration $r^8$ |
| 41 | 8 | 5 | $\sqrt{18}$ | $(56r^6-105r^4+60r^2-10)r^2\cos2\varphi$ | astigmatism $r^8$ 0°/90° |
| 42 | 8 | 6 | $\sqrt{18}$ | $(28r^4-42r^2+15)r^4\cos4\varphi$ | 4-fold $r^8$ along x |
| 43 | 8 | 7 | $\sqrt{18}$ | $(8r^2-7)r^6\cos6\varphi$ | 6-fold $r^8$ along x |
| 44 | 8 | 8 | $\sqrt{18}$ | $r^8\cos8\varphi$ | 8-fold along x |

## Table 2: Terms computed from the summation formulae of the trigonometric functions for n-fold angles

| $n$ | $\sin n\phi$ | $\cos n\phi$ |
|---|---|---|
| 2 | $\frac{1}{r^2}2xy$ | $\frac{1}{r^2}(x^2-y^2)$ |
| 3 | $\frac{1}{r^3}y(3x^2-y^2)$ | $\frac{1}{r^3}x(x^2-3y^2)$ |
| 4 | $\frac{1}{r^4}2xy(2x^2-2y^2)$ | $\frac{1}{r^4}(x^4-6x^2y^2+y^4)$ |
| 5 | $\frac{1}{r^5}y(5x^4-10x^2y^2+y^4)$ | $\frac{1}{r^5}x(x^4-10x^2y^2+5y^4)$ |
| 6 | $\frac{1}{r^6}2xy(3x^4-10x^2y^2+3y^4)$ | $\frac{1}{r^6}(x^6-15x^4y^2+15x^2y^4-y^6)$ |
| 7 | $\frac{1}{r^7}y(7x^6-35x^4y^2+21x^2y^4-y^8)$ | $\frac{1}{r^7}x(x^6-21x^4y^2+35x^2y^4-7y^6)$ |
| 8 | $\frac{1}{r^8}2xy(4x^6-28x^4y^2+28x^2y^4-4y^6)$ | $\frac{1}{r^8}(x^8-28x^6y^2+70x^4y^4-28x^2y^6+y^8)$ |

Table 3: Description of the order specified by the n Zernike polynomials

| Order n | Description | N |
|---|---|---|
| 1 | tilt | 2 |
| 2 | spherocyclic refraction | 5 |
| 3 | coma | 9 |
| 4 | spherical aberration | 14 |
| 5 | coma | 20 |
| 6 | spherical aberration | 27 |
| 7 | coma | 35 |
| 8 | spherical aberration | 44 |

## Table 4: The first 45 Zernike polynomials in Cartesian coordinates

| N | F | Polynome in kartesischen Koordinaten |
|---|---|---|
| 0 | 1 | $1$ |
| 1 | 2 | $y$ |
| 2 | 2 | $x$ |
| 3 | $\sqrt{6}$ | $2xy$ |
| 4 | $\sqrt{3}$ | $2(x^2 + y^2) - 1$ |
| 5 | $\sqrt{6}$ | $x^2 - y^2$ |
| 6 | $\sqrt{8}$ | $y(3x^2 - y^2)$ |
| 7 | $\sqrt{8}$ | $y[3(x^2 + y^2) - 2]$ |
| 8 | $\sqrt{8}$ | $x[3(x^2 + y^2) - 2]$ |
| 9 | $\sqrt{8}$ | $x(x^2 - 3y^2)$ |
| 10 | $\sqrt{10}$ | $4xy(x^2 - y^2)$ |
| 11 | $\sqrt{10}$ | $2xy(4(x^2 + y^2) - 3)$ |
| 12 | $\sqrt{5}$ | $6(x^2 + y^2)^2 - 6(x^2 + y^2) + 1$ |
| 13 | $\sqrt{10}$ | $(x^2 - y^2)[4(x^2 + y^2) - 3]$ |
| 14 | $\sqrt{10}$ | $x^4 - 6x^2y^2 + y^4$ |
| 15 | $\sqrt{12}$ | $y(5x^4 - 10x^2y^2 + y^4)$ |
| 16 | $\sqrt{12}$ | $y(3x^2 - y^2)[5(x^2 + y^2) - 4]$ |
| 17 | $\sqrt{12}$ | $y[10(x^2 + y^2)^2 - 12(x^2 + y^2) + 3]$ |
| 18 | $\sqrt{12}$ | $x[10(x^2 + y^2)^2 - 12(x^2 + y^2) + 3]$ |
| 19 | $\sqrt{12}$ | $x(x^2 - 3y^2)[5(x^2 + y^2) - 4]$ |
| 20 | $\sqrt{12}$ | $x(x^4 - 10x^2y^2 + 5y^4)$ |
| 21 | $\sqrt{14}$ | $2xy(3x^4 - 10x^2y^2 + 3y^4)$ |
| 22 | $\sqrt{14}$ | $4xy(x^2 - y^2)[6(x^2 + y^2) - 5]$ |
| 23 | $\sqrt{14}$ | $2xy[15(x^2 + y^2)^2 - 20(x^2 + y^2) + 6]$ |
| 24 | $\sqrt{7}$ | $20(x^2 + y^2)^3 - 30(x^2 + y^2)^2 + 12(x^2 + y^2) - 1$ |
| 25 | $\sqrt{14}$ | $(x^2 - y^2)[15(x^2 + y^2)^2 - 20(x^2 + y^2) + 6]$ |
| 26 | $\sqrt{14}$ | $(x^4 - 6x^2y^2 + y^4)[6(x^2 + y^2) - 5]$ |
| 27 | $\sqrt{14}$ | $x^6 - 15x^4y^2 + 15x^2y^4 - y^6$ |
| 28 | 4 | $y(7x^6 - 35x^4y^2 + 21x^2y^4 - y^6)$ |
| 29 | 4 | $y(5x^4 - 10x^2y^2 - y^4)[7(x^2 + y^2) - 6]$ |
| 30 | 4 | $y(3x^2 - y^2)[21(x^2 + y^2)^2 - 30(x^2 + y^2) + 10]$ |
| 31 | 4 | $y[35(x^2 + y^2)^3 - 60(x^2 - y^2)^2 + 30(x^2 + y^2) - 4]$ |
| 32 | 4 | $x[35(x^2 + y^2)^3 - 60(x^2 + y^2)^2 + 30(x^2 + y^2) - 4]$ |
| 33 | 4 | $x(x^2 - 3y^2)[21(x^2 + y^2)^2 - 30(x^2 + y^2) + 10]$ |
| 34 | 4 | $x(x^4 - 10x^2y^2 + 5y^4)[7(x^2 + y^2) - 6]$ |
| 35 | 4 | $x(x^6 - 21x^4y^2 + 35x^2y^4 - 7y^6)$ |
| 36 | $\sqrt{18}$ | $8xy(x^6 - 7x^4y^2 + 7x^2y^4 - y^6)$ |
| 37 | $\sqrt{18}$ | $2xy(3x^4 - 10x^2y^2 + 3y^4)[8(x^2 + y^2) - 7]$ |
| 38 | $\sqrt{18}$ | $4xy(x^2 - y^2)[28(x^2 + y^2)^2 - 42(x^2 + y^2) + 15]$ |
| 39 | $\sqrt{18}$ | $2xy[56(x^2 + y^2)^3 - 105(x^2 + y^2)^2 + 60(x^2 + y^2) - 10]$ |
| 40 | 3 | $70(x^2 + y^2)^4 - 140(x^2 + y^2)^3 + 90(x^2 + y^2)^2 - 20(x^2 + y^2) + 1$ |
| 41 | $\sqrt{18}$ | $(x^2 - y^2)[56(x^2 + y^2)^3 - 105(x^2 + y^2)^2 + 60(x^2 + y^2) - 10]$ |
| 42 | $\sqrt{18}$ | $(x^4 - 6x^2y^2 + y^4)[28(x^2 + y^2)^2 - 42(x^2 + y^2) + 15]$ |
| 43 | $\sqrt{18}$ | $(x^6 - 15x^4y^2 + 15x^2y^4 - y^6)[8(x^2 + y^2) - 7]$ |
| 44 | $\sqrt{18}$ | $x^8 - 28x^6y^2 + 70x^4y^4 - 28x^2y^6 + y^8$ |

**Table 5:** The partial derivatives with respect to x of the first 45 Zernike polynomials

| N | partielle Ableitungen nach x |
|---|---|
| 0 | 0 |
| 1 | 0 |
| 2 | 1 |
| 3 | $2y$ |
| 4 | $4x$ |
| 5 | $2x$ |
| 6 | $6xy$ |
| 7 | $6xy$ |
| 8 | $3(3x^2 + y^2) - 2$ |
| 9 | $3(x^2 - y^2)$ |
| 10 | $4y(3x^2 - y^2)$ |
| 11 | $2y[4(3x^2 + y^2) - 3]$ |
| 12 | $12x[2(x^2 + y^2) - 1]$ |
| 13 | $2x(8x^2 - 3)$ |
| 14 | $4x(x^2 - 3y^2)$ |
| 15 | $20xy(x^2 - y^2)$ |
| 16 | $4xy[5(3x^2 + y^2) - 6]$ |
| 17 | $8xy[5(x^2 + y^2) - 3]$ |
| 18 | $10(5x^4 + 6x^2y^2 + y^4) - 12(3x^2 + y^2) + 3$ |
| 19 | $5(5x^4 - 6x^2y^2 - 3y^4) - 12(x^2 - y^2)$ |
| 20 | $5(x^4 - 6x^2y^2 + y^4)$ |
| 21 | $6y(5x^4 - 10x^2y^2 + y^4)$ |
| 22 | $4y[6(5x^4 - y^4) - 5(3x^2 - y^2)]$ |
| 23 | $2y[15(5x^4 + 6x^2y^2 + y^4) - 20(3x^2 + y^2) - 6]$ |
| 24 | $24x[5(x^2 + y^2)^2 - 5(x^2 + y^2) + 1]$ |
| 25 | $2x[15(3x^4 + 2x^2y^2 - y^4) \cdot 40x^2 + 6]$ |
| 26 | $4x[3(3x^4 - 10x^2y^2 - 5y^4) - 5(x^2 - 3y^2)]$ |
| 27 | $6x(x^4 - 10x^2y^4 + 5y^4)$ |
| 28 | $14xy(3x^4 - 10x^2y^2 + 3y^4)$ |
| 29 | $2xy[120x^4 - 70x^2y^2 - 63y^4 - 60(x^2 - y^2)]$ |
| 30 | $6xy[7(9x^4 + 10x^2y^2 + y^4) - 20(3x^2 + y^2) + 10]$ |
| 31 | $30xy[7(x^2 + y^2)^2 - 8(x^2 + y^2) + 2]$ |
| 32 | $35(7x^6 + 15x^4y^2 + 9x^2y^4 + y^6) - 60(5x^4 + 6x^2y^2 + y^4) + 30(3x^2 + y^2) - 4$ |
| 33 | $21(7x^6 - 5x^4y^2 - 15x^2y^4 - 3y^6) - 30(5x^4 - 6x^2y^2 - 3y^4) + 30(x^2 - y^2)$ |
| 34 | $7(7x^6 - 45x^4y^2 - 15x^2y^4 + 5y^6) - 30(x^4 - 6x^2y^2 + y^4)$ |
| 35 | $7(x^6 - 15x^4y^2 + 15x^2y^4 - y^6)$ |
| 36 | $8y(7x^6 - 35x^4y^2 + 21x^2y^4 - y^6)$ |
| 37 | $2y[8(21x^6 - 35x^4y^2 - 21x^2y^4 + 3y^6) - 21(5x^4 - 10x^2y^2 + y^4)]$ |
| 38 | $4y[28(7x^6 + 5x^4y^2 - 3x^2y^4 - y^6) - 42(5x^4 - y^4) + 15(3x^2 - y^2)]$ |
| 39 | $2y[56(7x^6 + 15x^4y^2 + 9x^2y^4 + y^6) - 105(5x^4 + 6x^2y^2 + y^4) + 60(3x^2 + y^2) - 10]$ |
| 40 | $40x[14(x^2 + y^2)^3 - 21(x^2 + y^2)^2 + 9(x^2 + y^2) - 1]$ |
| 41 | $2x[112(2x^6 + 3x^4y^2 - y^6) - 105(3x^4 + 2x^2y^2 - y^4) + 10(12x^2 - 1)]$ |
| 42 | $4x[56(x^6 - 3x^4y^2 - 5x^2y^4 - y^6) - 21(3x^4 - 10x^2y^2 - 5y^4) - 15(x^2 - 3y^2)]$ |
| 43 | $2x[16(2x^6 - 21x^4y^2 + 7y^6) - 21(x^4 - 10x^2y^2 + 5y^4)]$ |
| 44 | $8x(x^6 - 21x^4y^2 + 35x^2y^4 - 7y^6)$ |

**Table 6:** The partial derivatives with respect to y of the first 45 Zernike polynomials

| N | partielle Ableitungen nach y |
|---|---|
| 0 | $0$ |
| 1 | $1$ |
| 2 | $0$ |
| 3 | $2x$ |
| 4 | $4y$ |
| 5 | $-2y$ |
| 6 | $3(x^2 - y^2)$ |
| 7 | $3(x^2 + 3y^2) - 2$ |
| 8 | $6xy$ |
| 9 | $-6xy$ |
| 10 | $4x(x^2 - 3y^2)$ |
| 11 | $2x[4(x^2 + 3y^2) - 3]$ |
| 12 | $12y[2(x^2 + y^2) - 1]$ |
| 13 | $2y(3 - 8y^2)$ |
| 14 | $4y(y^2 - 3x^2)$ |
| 15 | $5(x^4 - 6x^2y^2 + y^4)$ |
| 16 | $5(3x^4 + 6x^2y^2 - 5y^4) - 12(x^2 - y^2)$ |
| 17 | $10(x^4 - 6x^2y^2 + 5y^4) - 12(x^2 + 3y^2) + 3$ |
| 18 | $8xy[5(x^2 + y^2) - 3]$ |
| 19 | $-4xy[5(x^2 + 3y^2) - 6]$ |
| 20 | $-20xy(x^2 - y^2)$ |
| 21 | $6x(x^4 - 10x^2y^4 + 5y^4)$ |
| 22 | $-4x[6(x^4 - 5y^4) + 5(x^2 - 3y^2)]$ |
| 23 | $2x[15(x^4 + 6x^2y^2 + 5y^4) - 20(x^2 + 3y^2) - 6]$ |
| 24 | $24y[5(x^2 + y^2)^2 - 5(x^2 + y^2) + 1]$ |
| 25 | $-2y[15(x^4 + 2x^2y^2 - 3y^4) + 40y^2 - 6]$ |
| 26 | $4y[3(5x^4 - 10x^2y^2 - 3y^4) - 5(3x^2 - y^2)]$ |
| 27 | $-6y(5x^4 - 10x^2y^2 + y^4)$ |
| 28 | $7(x^6 - 15x^4y^2 + 15x^2y^4 - y^6)$ |
| 29 | $-7(7x^6 - 45x^4y^2 - 15x^2y^4 + 5y^6) + 30(x^4 - 6x^2y^2 + y^4)$ |
| 30 | $-21(3x^6 - 15x^4y^2 - 5x^2y^4 - 7y^6) + 30(3x^4 - 6x^2y^2 - 5y^4) + 30(x^2 - y^2)$ |
| 31 | $35(x^6 + 9x^4y^2 + 15x^2y^4 + 7y^6) - 60(x^4 + 6x^2y^2 + 5y^4) + 30(x^2 + 3y^2) - 4$ |
| 32 | $30xy[7(x^2 + y^2)^2 - 8(x^2 + y^2) + 2]$ |
| 33 | $-6xy[7(x^4 + 10x^2y^2 + 9y^4) - 20(x^2 + 3y^2) + 10]$ |
| 34 | $-2xy[120x^4 - 70x^2y^2 - 63y^4 - 60(x^2 - y^2)]$ |
| 35 | $-14xy(3x^4 - 10x^2y^2 + 3y^4)$ |
| 36 | $-8x(x^6 - 21x^3y^2 + 35x^2y^4 - 7y^6)$ |
| 37 | $2x[8(3x^6 - 21x^4y^2 - 35x^2y^4 - 21y^6) - 21(x^4 - 10x^2y^2 + 5y^4)]$ |
| 38 | $-4x[28(x^6 + 3x^4y^2 - 5x^2y^4 - 7y^6) - 42(x^4 - 5y^4) + 15(x^2 - 3y^2)]$ |
| 39 | $2x[56(x^6 + 9x^4y^2 + 15x^2y^4 + 7y^6) - 105(x^4 + 6x^2y^2 - 5y^4) + 60(x^2 + 3y^2) - 10]$ |
| 40 | $40y[14(x^2 + y^2)^3 - 21(x^2 + y^2)^2 + 9(x^2 + y^2) - 1]$ |
| 41 | $-2y[112(x^6 + 3x^2y^4 - 2y^6) - 105(x^4 + 2x^2y^2 - 3y^4) + 10(12y^2 - 1)]$ |
| 42 | $4y[56(x^6 - 5x^4y^2 - 3x^2y^4 - y^6) - 21(5x^4 - 10x^2y^2 - 3y^4) + 15(3x^2 - y^2)]$ |
| 43 | $-2y[16(7x^6 - 21x^2y^4 + 2y^6) - 21(5x^4 - 10x^2y^2 + y^4)]$ |
| 44 | $8y(7x^6 - 35x^4y^2 - 21x^2y^4 - y^6)$ |

List of Reference Symbols

| | |
|---|---|
| 10 | apparatus (aberrometer) |
| 12 | eye |
| 14 | light-source |
| 16 | bundle of light rays |
| 18 | component-beam-generating device |
| 19 | aperture mask |
| 20 | first optical axis (of the eye 12) |
| 22 | component beams |
| 22-1 - 22-5 | component beams |
| 23-1 | imaging-error-free component beam |
| 24 | first plane |
| 26 | two-dimensional arrangement |
| 27 | initial aperture |
| 28 | aberroscope lens |
| 29, $D_{29}$ | spacing between the aberroscope lens 28 and the optical system 30 of the eye |
| 30 | optical system (of the eye 12) |
| 32 | cornea |
| 34 | lens |
| 36 | focal point |
| 38 | predetermined distance |
| 40 | reti na |
| 50 | ophthalmoscopic device |
| 52 | second optical axis |
| 54 | second plane |
| 56 | first partially transmitting mirror |
| 58 | first ophthalmoscope lens |
| 60 | first recording device |
| 62 | CCD camera |
| 64 | diaphragm |
| 66 | optical channel |
| 68 | camera lens |
| 69 | deflecting mirror |
| 70 | laser |
| 73 | shutter device |
| 74 | laser beam |
| 76 | collimator device |
| 78 | Keplerian telescope |
| 80 | eye-aligning device |
| 82 | third optical axis |
| 84 | third plane |
| 86 | second partially transmitting mirror |
| 88 | second ophthalmoscope lens |
| 89 | IR lens |
| 90 | (second) recording device |
| 92 | IR CCD camera |
| 94 | IR band-pass filter |
| 98 | computer |
| 100 | wavefront aberration |
| 102 | Tscherning-aberrometry wavefront or real wavefront or other wavefront |
| 104 | wavefront(reference or target wavefront) |
| 110 | two-dimensional arrangement |
| 112 | component beams |
| 112-1, 112-2, 112-3, 112-4, 112-5 | component beams |
| 114 | first perpendicular plane (in the component-beam-generating device 18) |

| 116 | first pattern of dots (on the retina) |
| 117 | first projection of a component beam (on the retina) |
| 118 | second pattern of dots (projected ophthalmoscopically into the second plane) |
| 119 | second projection of a component beam (projected ophthalmoscopically into the second plane) |
| 120 | second plane |
| 124 | offset |
| 126 | offset in the x-direction |
| 128 | offset in the y-direction |
| 130 | aberration-free eye model |
| 132 | ideal point of light (projection of an aberration-free component beam) |
| 140 | Gullstrand eye model |
| 141 | front lens |
| 142 | first surface |
| 143 | rear lens |
| 144 | second surface |
| 146 | third surface |
| 148 | model retina |
| 149, $OL_{149}$ | model ocular length of the Gullstrand eye model |
| 150 | Liou-Brennan eye model |
| 151 | front lens |
| 152 | first surface |
| 154 | second surface |
| 155 | rear lens |
| 156 | third surface |
| 158 | fourth surface |
| 160 | fifth surface |
| 162 | model retina of the Liou-Brennan eye model |
| 164, $OL_{164}$ | model ocular length of the Liou-Brennan eye model |
| 172 | optical system of the individualised eye model |
| 173 | cornea |
| 174 | anterior corneal surface |
| 176 | posterior corneal surface |
| 178 | anterior chamber |
| 180 | lens |
| 182 | front lens face |
| 184 | back lens face |
| 186 | pupil of the individualised eye model |
| 187 | pupillary radius |
| 188 | model retina of the individualised eye model |
| 189 | ideal focal point (on the model retina 210) |
| 192 | corneal thickness, CT |
| 194 | anterior-chamber depth, ACD |
| 196 | lens thickness, LT |
| 198 | ocular length, OL |
| 200 | individual eye model |
| 202 | anterior corneal surface |
| 204 | posterior corneal surface |
| 206 | front lens face |
| 208 | back lens face |
| 210 | retina (model retina) |
| 212 | bundle of light rays (ray-tracing beam) |
| 216 | direction of a ray-tracing beam |
| 218 | reconstructed wavefront aberration (of the individualised eye model 200) |
| 220 | pre-operative anterior corneal surface (of the eye 12) |
| 222 | ideal post-operative anterior corneal surface |
| 224 | improved/optimised ablation profile |
| 230 | intraocular lens (IOL) |

| 232 | front lens face |
| 234 | intraocular wavefront upstream of the front lens face to be optimised |
| 235 | intraocular wavefront downstream of the front lens face to be optimised |
| 237 | back lens face |
| 238 | flat target wavefront |
| 240 | target wavefront with multifocal properties |

**Claims**

1. Process for determining optical aberrations of an eye (12), comprising:

   - measuring a length of said eye (12) to yield a measured ocular length (198, OL);
   - determining, using aberrometry, wavefront aberrations (100) of the eye (12) as a deviation of a wavefront (102) resulting from the optical system (30) of the eye **characterized in that** deviation is considered (12) with respect to a wavefront (104) generated by an aberration-free eye model that uses the measured ocular length (198, OL) for the model ocular length.

2. Process according to Claim 1, the determining step comprising:

   projecting an arrangement (26) of a plurality of component beams (22) through the optical system (30) of the eye (12) to yield a first pattern of dots (116) on the retina (40) of the eye;
   receiving a second pattern of dots (118) with a plurality of second projections (119) formed by projecting the first pattern of dots (116) onto a second plane (54); and
   comparing the second projections (119) with a plurality of model projections generated by the aberration-free eye model.

3. Process according to Claim 1 with the following steps:

   (a) generating a plurality of individual component beams (22; 22-1, ..., 22-5) of a bundle of light rays (16) of parallel light, the component beams (22) exhibiting a two-dimensional arrangement (26);
   (b) projecting the arrangement (26) of the component beams (22) through the optical system (30) of the eye (12) to yield a first pattern of dots (116) with first projections (117) of the component beams (22; 22-1, ... , 22-5) on the retina (40) of the eye;
   (c) ophthalmoscopic projecting of the first pattern of dots (116) onto a second plane (54) arranged outside of the eye (12) to yield a second pattern of dots (118) with second projections (119) of the first projections (117);
   (d) recording the second pattern of dots (118) on the second plane (54);
   (f) for each second projection (119), measuring an offset (124) of the ophthalmoscopic projection (119) of the component beam in the second pattern of dots (118) with respect to a projection (132), generated with the constructed eye model, of the same component beam (22) of the two-dimensional arrangement (26).

4. Process according to one of Claims 1 or 2, **characterised in that** the eye model is constructed with the measured ocular length (198, OL) on the basis of a known eye model, like the Gullstrand eye model or the Liou-Brennan eye model.

5. Process according to one of Claims 1 to 3, **characterised in that** the measured ocular length (198, OL) is determined from a direct ocular-length measurement of the eye (12).

6. Process according to one of Claims 1 to 4, the determining step further comprising:

   representing the wavefront (102) generated by the optical system (30) of the eye (12) as a sum of Zernike polynomials normalised to a unit circle and weighted with corresponding Zernike polynomial coefficients; and determining the wavefront aberrations by determining the Zernike polynomial coefficients.

7. Process according to Claim 5, **characterised in that** in the sum of the Zernike polynomials the Zernike polynomials are taken into account as far as the 6th order, and/or as far as the 8th order.

8. Process according to Claim 5 or 6, further comprising:

computing a spherical refraction of the eye (12), a cylindrical refraction of the eye (12) and an angle of an astigmatism of the eye (12) from the Zernike polynomial coefficients for the third, fourth and fifth Zernike polynomial of the second order and from the radius (187) of the pupil (186) of the eye (12).

9. Process according to one of the preceding claims, further comprising:

generating an eye-specific eye model (200) using the determine wavefront aberrations.

10. Process according to Claim 8, **characterised in that** the eye-specific eye model (200) is furthermore created on the basis of one or more of the following:

(1) a corneal thickness (192, CT), an anterior-chamber depth (194, ACD), a lens thickness (196, LT) and the actual ocular length (198, OL) of the eye (12), which in each instance have been determined from measurements of the eye (12),
(2) a topography of the anterior corneal surface (174) and a topography of the posterior corneal surface (176), which in each instance have been obtained from measurements carried out in respect of the eye (12),
(3) a front lens face (182) and a back lens face (184), which in each instance have been acquired from an iterative computation utilising an optical ray-tracing process and the data stated under points (1) and (2).

11. Process according to Claim 8 or 9, further comprising:

computing an ablation profile (224) for laser-surgical refraction correction according to the eye-specific eye model (200).

12. Process according to one of Claims 8 or 9, further comprising:

computing a front lens face (232) of an intraocular lens (230) according to the eye-specific eye model (200).

13. Apparatus for determining optical aberrations (100) of an eye (12) with its optical system (30) including the cornea (32, 173) and the lens (34, 180), with the following:

an aberrometer (10) for measuring the optical aberrations (100) of the eye (12), the aberrometer (10) designed to:

generate a plurality of component beams (22; 22-1, ..., 22-5) of a bundle of light rays (16) of parallel light, the component beams (22) exhibiting a two-dimensional arrangement (26),
project the arrangement (26) of the component beams (22) through the optical system (30) of the eye (12) onto the retina (40) of the eye (12) to yield a first pattern of dots (116) with first projections (117) of the component beams (22; 22-1,..., 22-5), and
ophthalmoscopically project the first pattern of dots (116) onto a second plane (54) outside of the eye (12) to yield a second pattern of dots (118) with second projections (119) of the first projections (117);

**characterised by**
an arithmetic unit (98) adapted to:

construct an aberration-free eye model that uses an actual ocular length (198, OL) for the model ocular length; and reconstruct wavefront aberrations (100) of the eye (12) as a deviation of the wavefront (102) resulting from the optical system (30) of the eye (12) with the aberrometer (10) with respect to a planar wavefront (104) generated by the eye model.

14. Apparatus according to Claim 12, **characterised in that** the aberrometer (10) includes:

a light-source (14) for generating a bundle of light rays (16) of parallel light,
a device (18) for generating a plurality of component beams (22; 22-1, ... , 22-5) from the bundle of light rays (16) with a two-dimensional arrangement (26) of the component beams (22),
an aberroscope lens (28) arranged on a first optical axis (20) for projecting the arrangement (26) of the component beams (22) through the optical system (30) of the eye (12) to yield the first pattern of dots (116) with first projections (117) of the component beams (22; 22-1, ... , 22-5) on the retina (40) of the eye (12),
an ophthalmoscopic device (50, 80) for ophthalmoscopically projecting of the first pattern of dots (116) into a second plane (54) arranged outside the eye (12) to yield a second pattern of dots (118) with second projections (119) of the first projections (117), and

a measuring device for each second projection (119) of a component beam (22) recorded with the ophthalmoscopic device (50), an offset (124) of the second projection (119) in the second plane (54) with respect to a projection (132), generated with the eye model, of the same component beam (22) of the two-dimensional arrangement (26).

**Patentansprüche**

1. Verfahren zum Bestimmen optischer Aberrationen eines Auges (12), das Folgendes umfasst:

   - Messen einer Länge des Auges (12), um eine gemessene Okularlänge (198, OL) zu liefern;
   - Bestimmen unter Verwendung der Aberrometrie von Wellenfrontaberrationen (100) des Auges (12) als eine Abweichung einer Wellenfront (102), die sich aus dem optischen System (30) des Auges (12) ergibt, **dadurch gekennzeichnet, dass** die Abweichung in Bezug auf eine Wellenfront (104) betrachtet wird, die durch ein aberrationsfreies Augenmodell erzeugt wird, das die gemessene Okularlänge (198, OL) als Modellokularlänge verwendet.

2. Verfahren nach Anspruch 1, wobei der Bestimmungsschritt Folgendes umfasst:

   Projizieren einer Anordnung (26) aus mehreren Komponentenstrahlenbündeln (22) durch das optische System (30) des Auges (12), um ein erstes Muster von Punkten (116) auf der Netzhaut (40) des Auges zu liefern;
   Empfangen eines zweiten Musters von Punkten (118) mit mehreren zweiten Projektionen (119), die durch Projizieren des ersten Musters von Punkten (116) auf eine zweite Ebene (54) gebildet werden; und
   Vergleichen der zweiten Projektionen (119) mit mehreren Modellprojektionen, die durch das aberrationsfreie Augenmodell erzeugt werden.

3. Verfahren nach Anspruch 1, das die folgenden Schritte umfasst:

   (a) Erzeugen mehrerer einzelner Komponentenstrahlenbündel (22; 22-1, ..., 22-5) eines Bündels von Lichtstrahlen (16) parallelen Lichts, wobei die Komponentenstrahlenbündel (22) eine zweidimensionale Anordnung (26) haben;
   (b) Projizieren der Anordnung (26) der Komponentenstrahlenbündel (22) durch das optische System (30) des Auges (12), um ein erstes Muster von Punkten (116) mit ersten Projektionen (117) der Komponentenstrahlenbündel (22; 22-1, ..., 22-5) auf der Netzhaut (40) des Auges zu liefern;
   (c) ophthalmoskopisches Projizieren des ersten Musters von Punkten (116) auf eine zweite Ebene (54), die außerhalb des Auges (12) angeordnet ist, um ein zweites Muster von Punkten (118) mit zweiten Projektionen (119) der ersten Projektionen (117) zu liefern;
   (d) Aufzeichnen des zweiten Musters von Punkten (118) auf die zweite Ebene (54);
   (f) für jede zweite Projektion (119) Messen eines Versatzes (124) der ophthalmoskopischen Projektion (119) des Komponentenstrahlenbündels in dem zweiten Muster von Punkten (118) in Bezug auf eine mit dem konstruierten Augenmodell erzeugte Projektion (132) desselben Komponentenstrahlenbündels (22) der zweidimensionalen Anordnung (26).

4. Verfahren nach einem der Ansprüche 1 oder 2, **dadurch gekennzeichnet, dass** das Augenmodell mit der gemessenen Okularlänge (198, OL) auf der Grundlage eines bekannten Augenmodells wie etwa des Gullstrand-Augenmodells oder des Liou-Brennan-Augenmodells konstruiert wird.

5. Verfahren nach einem der Ansprüche 1 bis 3, **dadurch gekennzeichnet, dass** die gemessenen Okularlänge (198, OL) aus einer direkten Okularlängenmessung des Auges (12) bestimmt wird.

6. Verfahren nach einem der Ansprüche 1 bis 4, wobei der Bestimmungsschritt ferner Folgendes umfasst:

   Darstellen der Wellenfront (102), die durch das optische System (30) des Auges (12) erzeugt wird, als eine Summe von Zernike-Polynomen, die auf einen Einheitskreis normiert sind und mit entsprechenden Zernike-Polynomkoeffizienten erzeugt wird; und
   Bestimmen der Wellenfrontaberrationen durch Bestimmen der Zernike-Polynomkoeffizienten.

7. Verfahren nach Anspruch 5, **dadurch gekennzeichnet, dass** in der Summe der Zernike-Polynome die Zernike-Polynome bis zur 6. Ordnung und/oder bis zur 8. Ordnung berücksichtigt werden.

8. Verfahren nach Anspruch 5 oder 6, das ferner Folgendes umfasst:

Berechnen einer sphärischen Brechung des Auges (12), einer zylindrischen Brechung des Auges (12) und eines Astigmatismuswinkels des Auges (12) aus den Zernike-Polynomkoeffizienten für das dritte, vierte und fünfte Zernike-Polynom zweiter Ordnung und aus dem Radius (187) der Pupille (186) des Auges (12).

9. Verfahren nach einem der vorhergehenden Ansprüche, das ferner Folgendes umfasst:

Erzeugen eines augenspezifischen Augenmodells (200) unter Verwendung der bestimmten Wellenfrontaberrationen.

10. Verfahren nach Anspruch 8, **dadurch gekennzeichnet, dass** das augenspezifische Augenmodell (200) ferner auf der Grundlage eines oder mehrerer der Folgenden erzeugt wird:

(1) einer Hornhautdicke (192, CT), einer Vorderkammertiefe (194, ACD), einer Linsendicke (196, LT) und der tatsächlichen Okularlänge (198, OL) des Auges (12), die in jedem Fall aus Messungen des Auges (12) bestimmt worden sind,
(2) einer Topographie der vorderen Hornhautoberfläche (174) und einer Topographie der hinteren Hornhautoberfläche (176), die in jedem Fall aus Messungen erhalten worden sind, die in Bezug auf das Auge (12) ausgeführt werden,
(3) einer vorderen Linsenfläche (182) und einer hinteren Linsenfläche (184), die in jedem Fall aus einer iterativen Berechnung unter Verwendung eines Lichtstrahlverfolgungsprozesses und der unter den Punkten (1) und (2) angegebenen Daten erfasst worden sind.

11. Verfahren nach Anspruch 8 oder 9, das ferner Folgendes umfasst:

Berechnen eines Ablationsprofils (224) für eine laserchirurgische Brechungskorrektur in Übereinstimmung mit dem augenspezifischen Augenmodell (200).

12. Verfahren nach einem der Ansprüche 8 oder 9, das ferner Folgendes umfasst:

Berechnen einer vorderen Linsenfläche (232) einer Intraokularlinse (230) in Übereinstimmung mit dem augenspezifischen Augenmodell (200).

13. Vorrichtung zum Bestimmen optischer Aberrationen (100) eines Auges (12) mit seinem optischen System (30) einschließlich der Hornhaut (32, 173) und der Linse (34, 180) mit den folgenden Elementen:

einem Aberrometer (10) zum Messen der optischen Aberrationen (100) des Auges (12), wobei das Aberrometer (10) entworfen ist:

mehrere Komponentenstrahlenbündel (22; 22-1, ..., 22-5) eines Bündels von Lichtstrahlen (16) parallelen Lichts zu erzeugen, wobei die Komponentenstrahlenbündel (22) eine zweidimensionale Anordnung (26) haben,
die Anordnung (26) der Komponentenstrahlenbündel (22) durch das optische System (30) des Auges (12) auf die Netzhaut (40) des Auges (12) zu projizieren, um ein erstes Muster von Punkten (116) mit ersten Projektionen (117) der Komponentenstrahlenbündel (22; 22-1, ..., 22-5) zu liefern, und
das erste Muster von Punkten (116) ophthalmoskopisch auf eine zweite Ebene (54) außerhalb des Auges (12) zu projizieren, um ein zweites Muster von Punkten (118) mit zweiten Projektionen (119) der ersten Projektionen (117) zu liefern;

**gekennzeichnet durch**

eine Arithmetikeinheit (98), die dafür ausgelegt ist:

ein aberrationsfreies Augenmodell zu konstruieren, das eine tatsächliche Okularlänge (198, OL) als Modellokularlänge verwendet; und

Wellenfrontaberrationen (100) des Auges (12) als eine Abweichung der Wellenfront (102), die sich aus dem optischen System (30) des Auges (12) ergibt, mit dem Aberrometer (10) in Bezug auf eine ebene Wellenfront (104), die **durch** das Augenmodell erzeugt wird, zu rekonstruieren.

**14.** Vorrichtung nach Anspruch 12, **dadurch gekennzeichnet, dass** das Aberrometer (10) Folgendes umfasst:

eine Lichtquelle (14) zum Erzeugen eines Bündels von Lichtstrahlen (16) parallelen Lichts,

eine Einrichtung (18) zum Erzeugen mehrerer Komponentenstrahlenbündel (22; 22-1, ..., 22-5) aus dem Bündel von Lichtstrahlen (16) mit einer zweidimensionalen Anordnung (26) der Komponentenstrahlenbündel (22),

eine Aberroskop-Linse (28), die auf einer ersten optischen Achse (20) angeordnet ist, um die Anordnung (26) der Komponentenstrahlenbündel (22) durch das optische System (30) des Auges (12) zu projizieren, um das erste Muster von Punkten (116) mit ersten Projektionen (117) der Komponentenstrahlenbündel (22; 22-1, ..., 22-5) auf der Netzhaut (40) des Auges (12) zu liefern,

eine ophthalmoskopische Einrichtung (50, 80), um das erste Muster von Punkten (116) ophthalmoskopisch in eine zweite Ebene (54), die außerhalb des Auges (12) angeordnet ist, zu projizieren, um ein zweites Muster von Punkten (118) mit zweiten Projektionen (119) der ersten Projektionen (117) zu liefern, und

eine Messeinrichtung für jede zweite Projektion (119) eines Komponentenstrahlenbündels (22), die auf die ophthalmoskopische Einrichtung (50) aufgezeichnet ist, eines Versatzes (124) der zweiten Projektion (119) in der zweiten Ebene (54) in Bezug auf eine Projektion (132), die mit dem Augenmodell erzeugt wird, desselben Komponentenstrahlenbündels (22) der zweidimensionalen Anordnung (26).

**Revendications**

**1.** Procédé de détermination d'aberrations optiques d'un oeil (12), consistant à :

- mesurer une longueur dudit oeil (12) afin d'obtenir une longueur oculaire mesurée (198, OL)) ;
- déterminer, par aberrométrie, des aberrations de front d'onde (100) de l'oeil (12) sous la forme d'un écart d'un front d'onde (102) résultant du système optique (30) de l'oeil (12), **caractérisé en ce que** l'écart est considéré par rapport à un front d'onde (104) généré par un modèle d'oeil dépourvu d'aberration qui utilise la longueur oculaire mesurée (198, OL) pour la longueur oculaire de modèle.

**2.** Procédé selon la revendication 1, l'étape de détermination consistant à :

projeter un agencement (26) d'une pluralité de faisceaux constitutifs (22) à travers le système optique (30) de l'oeil (12) afin d'obtenir un premier motif de points (112) sur la rétine (40) de l'oeil ;

recevoir un deuxième motif de points (118) au moyen d'une pluralité de deuxièmes projections (119) formées en projetant le premier motif de points (116) sur un deuxième plan (54) ; et

comparer les deuxièmes projections (119) à une pluralité de projections de modèle générées par le modèle d'oeil dépourvu d'aberration.

**3.** Procédé selon la revendication 1, comprenant les étapes suivantes :

(a) générer une pluralité de faisceaux constitutifs individuels (22 ; 22-1, ..., 22-5) d'un pinceau de rayons lumineux (16) de lumière parallèle, les faisceaux constitutifs (22) présentant un agencement bidimensionnel (26) ;

(b) projeter l'agencement (26) des faisceaux constitutifs (22) à travers le système optique (30) de l'oeil (12) afin d'obtenir un premier motif de points (116) au moyen de premières projections (117) des faisceaux constitutifs (22 ; 22-1, ..., 22-5) sur la rétine (40) de l'oeil ;

(c) projeter de manière ophtalmoscopique le premier motif de points (116) sur un deuxième plan (54) agencé à l'extérieur de l'oeil (12) afin d'obtenir un deuxième motif de points (118) au moyen de deuxièmes projections (119) des premières projections (117) ;

(d) enregistrer le deuxième motif de points (118) sur le deuxième plan (54) ;

(f) pour chaque deuxième projection (119), mesurer un décalage (124) de la projection ophtalmoscopique (119) du faisceau constitutif dans le deuxième motif de points (118) par rapport à une projection (132), générée au moyen du modèle d'oeil construit, du même faisceau constitutif (22) de l'agencement bidimensionnel (26).

**4.** Procédé selon la revendication 1 ou 2, **caractérisé en ce que** le modèle d'oeil est construit au moyen de la longueur oculaire mesurée (198, OL) sur la base d'un modèle d'oeil connu, tel que le modèle d'oeil de Gullstrand ou que le modèle d'oeil de Liou-Brennan.

5. Procédé selon l'une des revendications 1 à 3, **caractérisé en ce que** la longueur oculaire mesurée (198, OL) est déterminée par une mesure de longueur oculaire directe de l'oeil (12).

6. Procédé selon l'une des revendications 1 à 4, l'étape de détermination consistant en outre à :

représenter le front d'onde (102) généré par le système optique (30) de l'oeil (12) sous la forme d'une somme de polynômes de Zernike normalisés par rapport à un cercle unité et pondérés par des coefficients des polynômes de Zernike correspondants ; et
déterminer les aberrations de front d'onde en déterminant les coefficients des polynômes de Zernike.

7. Procédé selon la revendication 5, **caractérisé en ce que**, dans la somme de polynômes de Zernike, les polynômes de Zernike sont pris en compte jusqu'au 6ème ordre et/ou jusqu'au 8ème ordre.

8. Procédé selon la revendication 5 ou 6, consistant en outre à :

calculer une réfraction sphérique de l'oeil (12), une réfraction cylindrique de l'oeil (12) et un angle d'astigmatisme de l'oeil (12) à partir des coefficients des polynômes de Zernike pour les troisième, quatrième et cinquième polynômes de Zernike du deuxième ordre et à partir du rayon (187) de la pupille (186) de l'oeil (12).

9. Procédé selon l'une des revendications précédentes, consistant en outre à :

générer un modèle d'oeil spécifique de l'oeil (200) en utilisant les aberrations de front d'onde déterminées.

10. Procédé selon la revendication 8, **caractérisé en ce que** le modèle d'oeil spécifique de l'oeil (200) est en outre créé sur la base d'un ou plusieurs des éléments suivantes :

(1) une épaisseur cornéenne (192, CT), une profondeur de chambre antérieure (194, ACD), une épaisseur de cristallin (196, LT) et la longueur oculaire effective (198, OL) de l'oeil (12), qui ont été déterminées dans chaque cas à partir de mesures de l'oeil (12),
(2) une topographie de la surface cornéenne antérieure (174) et une topographie de la surface cornéenne postérieure (176) qui, dans chaque cas, ont été obtenues à partir de mesures effectuées sur l'oeil (12),
(3) une face de cristallin avant (182) et une face de cristallin arrière (184) qui, dans chaque cas, ont été acquises au moyen d'un calcul itératif utilisant un procédé de tracé de rayons optique et les données indiquées aux points (1) et (2).

11. Procédé selon la revendication 8 ou 9, consistant en outre à :

calculer un profil d'ablation (224) pour une correction de réfraction par chirurgie laser conformément au modèle d'oeil spécifique de l'oeil (200).

12. Procédé selon l'une des revendications 8 ou 9, consistant en outre à :

calculer une face de cristallin avant (232) d'un cristallin intraoculaire (230) conformément au modèle d'oeil spécifique de l'oeil (200).

13. Appareil permettant de déterminer des aberrations optiques (100) d'un oeil (12) au moyen de son système optique (30), comportant la cornée (32, 173) et le cristallin (34, 180), comprenant les éléments suivantes :

un aberromètre (10) permettant de mesurer les aberrations optiques (100) de l'oeil (12), l'aberromètre (10) étant conçu pour :

générer une pluralité de faisceaux constitutifs individuels (22 ; 22-1, ..., 22-5) d'un pinceau de rayons lumineux (16) de lumière parallèle, les faisceaux constitutifs (22) présentant un agencement bidimensionnel (26),
projeter l'agencement (26) des faisceaux constitutifs (22) à travers le système optique (30) de l'oeil (12) sur la rétine (40) de l'oeil (12) afin d'obtenir un premier motif de points (116) au moyen de premières projections (117) des faisceaux constitutifs (22 ; 22-1, ..., 22-5), et

projeter de manière ophtalmoscopique le premier motif de points (116) sur un deuxième plan (54) agencé à l'extérieur de l'oeil (12) afin d'obtenir un deuxième motif de points (118) au moyen de deuxièmes projections (119) des premières projections (117) ;

**caractérisé par**

une unité arithmétique (98) conçue pour :

construire un modèle d'oeil dépourvu d'aberration qui utilise une longueur oculaire effective (198, OL) pour la longueur oculaire de modèle ; et

reconstruire des aberrations de front d'onde (100) de l'oeil (12) sous la forme d'un écart du front d'onde (102) résultant du système optique (30) de l'oeil (12) au moyen de l'aberromètre (10) par rapport à un front d'onde plan (104) généré par le modèle d'oeil.

14. Appareil selon la revendication 12, **caractérisé en ce que** l'aberromètre (10) comporte :

une source lumineuse (14) permettant de générer un pinceau de rayons lumineux (16) de lumière parallèle,

un dispositif (18) permettant de générer une pluralité de faisceaux constitutifs (22 ; 22-1, ..., 22-5) à partir du pinceau de rayons lumineux (16) au moyen d'un agencement bidimensionnel (26) des faisceaux constitutifs (22),

une lentille d'aberroscope (28) agencée sur le premier axe optique (20) pour projeter l'agencement (26) des faisceaux constitutifs (22) à travers le système optique (30) de l'oeil (12) afin d'obtenir le premier motif de points (116) au moyen de premières projections (117) des faisceaux constitutifs (22 ; 22-1, ..., 22-5) sur la rétine (40) de l'oeil (12),

un dispositif ophtalmoscopique (50, 80) permettant de projeter de manière ophtalmoscopique le premier motif de points (116) sur un deuxième plan (54) agencé à l'extérieur de l'oeil (12) afin d'obtenir un deuxième motif de points (118) au moyen de deuxièmes projections (119) des premières projections (117), et

un dispositif de mesure pour chaque deuxième projection (119) d'un faisceau constitutif (22) enregistré au moyen du dispositif ophtalmoscopique (50), un décalage (124) de la deuxième projection (119) dans le deuxième plan (54) par rapport à une projection (132), générée au moyen du modèle d'oeil, du même faisceau constitutif (22) de l'agencement bidimensionnel (26).

FIG 1

54, 60

58    52

22-1

20, 52

22-2

18

22-3    20

22

22-4

22-5

28

56    30

D29, 29

198, OL

12

117

188, 40

116

36

38

198, OL

FIG 2

FIG 3

$54 \hat{=} 40 \times V$

$124 = (H40 - H040) \times V$

# FIG 4

| Surface | Radius (mm) | Thickness (mm) | Refraction index |
|---------|-------------|----------------|------------------|
| 1 = 142 | 7.8 | 3.6 | 1.336 |
| 2 = 144 | 10.0 | 3.6 | 1.413 |
| 3 = 146 | -6.0 | 16.97 | 1.336 |

## FIG 5

| Surface | Radius (mm) | Thickness (mm) | Asphericity | Refraction index (555 nm) |
|---------|-------------|----------------|-------------|---------------------------|
| 1 = 152 | 7.77 | 0.50 | -0.18 | 1.376 |
| 2 = 154 | 6.40 | 3.16 | -0.60 | 1.336 |
| 3 = 156 | 12.40 | 1.59 | -0.94 | Grad A |
| 4 = 158 | Infinity | 2.43 | — | Grad P |
| 5 = 160 | -8.10 | 16.27 | 0.96 | 1.336 |

$GradA = 1.368 + 0.049057 \cdot z - 0.015427 \cdot z^2 - 0.001978 \cdot r^2$    $GradP = 1.407 - 0.006605 \cdot z^2 - 0.001978 \cdot r^2$

$n(\lambda) = n(0.555\mu m) + 0.0512 - 0.1455 \cdot \lambda + 0.0961 \cdot \lambda^2$

EP 2 872 030 B1

FIG 6

# FIG 7

# FIG 8

FIG 9

# FIG 10

# FIG 11

FIG 12

220

220

220

222

222

222

224

224

224

FIG 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2003117581 A